# EUROPEAN PATENT SPECIFICATION

(11) **EP 3 611 504 B1**
(45) Date of publication and mention of the grant of the patent: **29.12.2021**
(21) Application number: 18784710.8
(22) Date of filing: 10.04.2018
(51) Int. Cl.: G01N 33/50, C12M 1/34

(54) **METHOD FOR DETECTING EFFECTOR-TYPE T-CELLS, T-CELLS ANALYZING DEVICE AND ANALYZING SYSTEM**
VERFAHREN ZUM NACHWEIS VON REGULATORISCHEN T-ZELLEN DES EFFEKTORTYPS, VORRICHTUNG UND SYSTEM ZUR ANALYSE VON T-ZELLEN
PROCÉDÉ DE DÉTECTION DE LYMPHOCYTES RÉGULATEURS DE TYPE EFFECTEUR, DISPOSITIF ET SYSTÈME D'ANALYSE DE LYMPHOCYTES T

(30) Priority: 14.04.2017 JP 2017080955
(43) Date of publication of application: 19.02.2020
(73) Proprietor: National Cancer Center Japan, Chuo-ku, Tokyo 104-0045 (JP); Sysmex Corporation, Kobe-shi, Hyogo 651-0073 (JP)
(72) Inventor: NISHIKAWA, Hiroyoshi, Kashiwa-shi Chiba 277-8577 (JP); KOBAYASHI, Tamiyo, Kobe-shi Hyogo 651-0073 (JP)
(74) Representative: Hoffmann Eitle
(86) International application number: PCT/JP2018/015094
(87) International publication number: WO 2018/190340

(56) References cited:
- EP-A1- 3 037 816
- WO-A1-2007/014420
- WO-A1-2016/010114
- JP-A- 2016 532 865
- SANTEGOETS SASKIA J ET AL: "Monitoring regulatory T cells in clinical samples: consensus on an essential marker set and gating strategy for regulatory T cell analysis by flow cytometry", CANCER IMMUNOLOGY, IMMUNOTHERAPY, NIH AUTHOR MANUSCRIPT, SPRINGER, BERLIN/HEIDELBERG, vol. 64, no. 10, 28 June 2015 (2015-06-28) , pages 1271-1286, XP035533182, ISSN: 0340-7004, DOI: 10.1007/S00262-015-1729-X [retrieved on 2015-06-28]
- SCHMIDT ANGELIKA ET AL: "Analysis of FOXP3+regulatory T cell subpopulations in peripheral blood and tissue of patients with systemic lupus erythematosus", IMMUNOLOGIC RESEARCH, HUMANA PRESS, INC, US, vol. 65, no. 2, 21 February 2017 (2017-02-21), pages 551-563, XP036236796, ISSN: 0257-277X, DOI: 10.1007/S12026-017-8904-4 [retrieved on 2017-02-21]
- H. FUJII ET AL: "Perturbations of both nonregulatory and regulatory FOXP3+ T cells in patients with malignant melanoma : FOXP3+ T-cell subsets in melanoma", BRITISH JOURNAL OF DERMATOLOGY, vol. 164, no. 5, 5 April 2011 (2011-04-05) , pages 1052-1060, XP055735183, UK ISSN: 0007-0963, DOI: 10.1111/j.1365-2133.2010.10199.x
- HIROKO FUJII ET AL: "Regulatory T Cells in Melanoma Revisited by a Computational Clustering of FOXP3 + T Cell Subpopulations", THE JOURNAL OF IMMUNOLOGY, vol. 196, no. 6, 10 February 2016 (2016-02-10), pages 2885-2892, XP055734431, US ISSN: 0022-1767, DOI: 10.4049/jimmunol.1402695
- W.Sun ET AL: "CD45RA-FOXP3high but not....", J. Experim. Clin. Cancer Res., vol. 33-35 31 December 2014 (2014-12-31), pages 1-10, XP055735189, DOI: 10.1109/TPEL.2011.2179121 Retrieved from the Internet: URL:www.jeccr.biomedcentral.com/articles/1 0.1186/1756-9966-33-35#Fig1 [retrieved on 2020-09-29]
- MAKOTO MIYARA ET AL: "Functional Delineation and Differentiation Dynamics of Human CD4+ T Cells Expressing the FoxP3 Transcription Factor", IMMUNITY, vol. 30, no. 6, 1 June 2009 (2009-06-01), pages 899-911, XP055479615, ISSN: 1074-7613, DOI: 10.1016/j.immuni.2009.03.019
- Nishikawa, Hakuyoshi: "Criteria of selective adaptation of cancer immunotherapy on the basis of novel immunological classification of colorectal cancer", Research Performance Report on Scientific Research Funds Raising Business, 10 June 2016 (2016-06-10), pages 1-6, XP009516771,
- HIRAHARA, K. et al.: "The Majority of Human Peripheral Blood CD 4+ CD 25hlgh Foxp3+ Regulatory T Cells Bear Functional Skin-Homing Receptors", Journal of Immunology, vol. 177, 2006, pages 4488-4494, XP055233375, DOI: 10.4049/jimmunol.177.7.4488
- WARD, S. T. et al.: "A method for conducting suppression assays using small numbers of tissue-isolated regulatory T cells", MethodsX, vol. 1, 1 January 2014 (2014-01-01), pages 168-174, XP055632527, ISSN: 2215-0161, DOI: 10.1016/j.mex.2014.08.012

## Description

### Technical Field

The present invention relates to an effector type regulatory T cell detecting method, an effector type regulatory T cell analyzing device, an effector type regulatory T cell analyzing system, and a program.

### Background Art

CD4-positive regulatory T cells (Treg) are T cells which play a central role in suppressing immune response. Among CD4-positive T cells, T cells expressing an IL-2 receptor α chain CD25 and a regulatory T cell transcription factor FOXP3 are defined as regulatory T cells. Furthermore, regulatory T cells are classified into naive types, effector types, and types without immunosuppressive activity (inactive type) depending on an expression level of CD45RA and an expression level of FOXP3. Among them, effector type regulatory T cells (eTreg) are known to have a strong immunosuppressive activity and to be deeply involved in induction of anticancer immunity. Thus, further analysis of the effector type regulatory T cells is expected in fields such as autoimmunity and anticancer immunity.

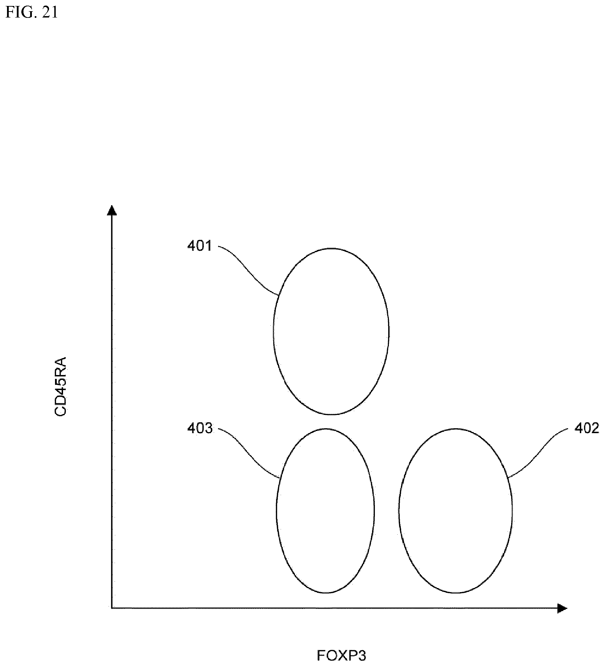

The following Non Patent Document 1 describes that the regulatory T cells can be classified into naive types, effector types, and inactive types by an analysis using a flow cytometer. Herein, each of CD45RA and FOXP3 is fluorescently labeled, and a scattergram presented in FIG. 21 is constructed from detection results according to the flow cytometer. In this scattergram, regulatory T cells included in regions 401, 402, and 403 are classified into the naive types, the effector types, and the inactive types, respectively.

Santegoets Saskia et al : "Monitoring regulatory T cells in clinical samples: consensus on an essential marker set and gating strategy for regulatory T cell analysis by flow cytometry", 28 June 2015, Springer, discloses the testing of proposed Treg markers to get insight into the overlap/differences between the most frequently used Treg definitions and their utility for Treg detection in various human tissues. Here, the authors conclude that the CD3, CD4, CD25, CD127, and FoxP3 markers are the minimally required markers to define human Treg cells.

Schmidt Angelika et al: "Analysis of F0XP3+regulatory T cell subpopulations in peripheral blood and tissue of patients with systemic lupus erythematosus", 21 February 2017, Humana Press, relates to regulatory T cells (Tregs) and their involvement in the autoimmune disease systemic lupus erythematosus (SLE).

H. Fuiji et al, "Perturbations of both nonregulatory and regulatory F0XP3+ T cells in patients with malignant melanoma: F0XP3+ T-cell subsets in melanoma", 5 April 2011, British Journal of Dermatology, discloses a method in which FOXP3+ T cells are classified into three subsets according to their expression levels of FOXP3 and CD45RO.

Hiroko Fujii et al. "Regulatory T Cells in Melanoma Revisited by a Computational Clustering of F0XP3 + T Cell Subpopulations", 10 February 2016, The journal of immunology, relates to regulatory T cells in melanoma. By analysing flow cytometric data of melanoma patients, the proposed method shows that the FOXP3+ subpopulation that had relatively high FOXP3, CD45RO, and CD25 expressions was increased in melanoma patients, whereas manual gating did not produce significant results on the FOXP3+ subpopulations.

W. Sun et al. "CD45RA-F0XP3high but not CD45RA+Foxp3low suppressive T regulatory cells increased in the peripheral circulation of patients with head and neck squamous cell carcinoma and correlated with tumor progression", 31 December 2014, describes a method in which, using multicolor flow cytometry, the frequency of three Treg subsets, separated on the basis of CD45RA and Foxp3, from the peripheral circulation of newly-presenting HNSCC patients were assessed with regard to 31 healthy donors and clinicopathological features.

EP 3 037 816 A1 discloses a method for determination of the frequency of regulatory T-cells in samples obtained from human blood and to methods for the preparation of compositions comprising predetermined amount of regulatory T-cells. The invention is based on the conception that a large fraction of regulatory T-cells present in human peripheral blood do not express detectable amounts of Foxp3, the master transcription factor used for identification of regulatory T-cells, as a result of cytokine deprivation outside of the tissue context.

WO 2007/014420 A1 relates to methods and kits for diagnosing or monitoring autoimmune diseases, immunoinflammatory diseases, allergic diseases, predispositions thereto, infectious diseases, cancer, cancer treatment and/or organ transplantation based on regulatory T cell quantity, to methods and kits for predicting responses to therapy for autoimmune diseases, immunoinflammatory diseases, allergic diseases, predispositions thereto, infectious diseases, cancer and/or organ transplantation based on regulatory T cell quantity, and to methods and kits for therapy using isolated regulatory T cells.

### Citation List

### Non Patent Literature

Non Patent Literature 1: Hiroyoshi Nishikawa and one other member, "RegulatoryT cells in cancer immunotherapy", Current Opinion in Immunology, 2014, Volume 27, p. 1-7

### Summary of Invention

### Technical Problem

However, in some samples, regions of the naive types, the effector types, and inactive types are indistinct, and the boundaries among these types may not be appropriately set. Thus, for such samples, effector type regulatory T cells could not have been detected with high accuracy.

In light of the above problems, the object of the present invention is to provide an effector type regulatory T cell detecting method, an effector type regulatory T cell analyzing device, an effector type regulatory T cell analyzing system and a program capable of detecting the effector type regulatory T cells with high accuracy.

### Solution to Problem

A first aspect of the present invention relates to an effector type regulatory T cell detecting method using flow cytometry. In the effector type regulatory T cell detecting method according to this aspect, a control sample containing a CD4-positive T cell population is measured, and based on a result of measuring the control sample, a FOXP3 level being a division reference for dividing a FOXP3-positive T cell population contained in the CD4-positive T cell population at a predetermined ratio is acquired as a reference value to be used for measuring a biological sample (S13). A biological sample containing a CD4-positive T cell population is measured (S22), and based on a result of measuring the biological sample, effector type regulatory T cells are detected from a cell population having a FOXP3 level higher than the reference value (S23).

The control sample refers to a sample measured for acquiring a reference value used for measuring the biological sample, and includes e.g. a peripheral blood mononuclear cell population equivalent to that of a healthy human. The control sample includes a wide range of samples or the like containing a peripheral blood itself of a healthy human, peripheral blood mononuclear cells purified from a peripheral blood of a healthy human by a density gradient method. The FOXP3 level refers to a value indicating what degree of the FOXP3 is contained in a cell. The healthy human refers to a person who does not suffer from a certain chronic disease and has no difficulty in daily life.

The effector type regulatory T cell detecting method according to this aspect allows uniform setting of a reference value, because the reference value is set by dividing the FOXP3-positive T cell population acquired by measuring the control sample at a predetermined ratio. In addition, as described in the following embodiment, the reference value can be appropriately set by using the control sample even if a measurement environment changes. Furthermore, since the reference value has been acquired by previously measuring the control sample, a reference value corresponding to the measurement environment can be set as a reference value for measuring the biological sample. As described above, since the detection method according to this aspect allows uniform and appropriate setting of the reference value used for measuring the biological sample, the effector type regulatory T cells can be stably detected with high accuracy.

In the effector type regulatory T cell detecting method according to this aspect, the control sample is measured for each measurement of one biological sample to acquire the reference value, and the acquired reference value is used for detecting the effector type regulatory T cells in the measurement of one biological sample. In this way, the reference value is set for each measurement of the biological sample, and therefore a reference value adapted to a measurement environment for measuring the biological sample can be set. Thus, the detection accuracy for the effector type regulatory T cells can be increased.

In the effector type regulatory T cell detecting method according to this aspect, the control sample is measured for each measurement of a plurality of biological samples to acquire the reference value, and the acquired reference value is used for detecting the effector type regulatory T cells in the measurement of each biological sample. In this way, a measurement frequency for the control sample can be reduced, and therefore a measurement efficiency for the biological samples can be enhanced. When the plurality of biological samples can be prepared and measured under uniform conditions, this method is particularly effective, like a case that the biological samples are automatically prepared and measured by a device. In addition, since a state of a reagent may be changed at a timing of changing a reagent lot, it is preferable to set a reference value with the control sample again, even when the biological samples are automatically prepared and measured by the device.

In the effector type regulatory T cell detecting method according to this aspect, the reference value is acquired by applying the ratio to an entire FOXP3-positive T cell population acquired based on the result of measuring the control sample (S108).

In this case, the ratio is set so that a proportion of a number of cells having a higher FOXP3 level to a total cell number of the FOXP3-positive T cell population acquired based on the result of measuring the control sample is 57% or more to 72% or less. The ratio is set in such a way, so that the reference value can be appropriately set as described in the following embodiment.

In addition, the ratio may be set so that a ratio of a number of cells having the higher FOXP3 level relative to a number of cells having a lower FOXP3 level is 1.35 or more to 2.48 or less in the FOXP3-positive T cell population acquired based on the result of measuring the control sample. The ratio is set in such a way, so that the reference value can be appropriately set as described in the following embodiment.

In the effector type regulatory T cell detecting method according to this aspect, when the CD4-positive T cell population acquired based on the result of measuring the control sample is represented in a histogram (71) with an axis of the FOXP3 level, a cell population having a FOXP3 level equal to or higher than a FOXP3 level at an end point of a peak waveform in a FOXP3-negative side of the histogram (71) is specified as a FOXP3-positive T cell population (S107). In this way, the FOXP3-positive T cell population can be appropriately specified.

In this case, when an approximated curve is applied to the peak waveform in the FOXP3-negative side of the histogram (71), a point at which the approximated curve intersects with the axis in a higher value side of the FOXP3 level is set as the end point. In this way, the end point can be uniformly set.

In the effector type regulatory T cell detecting method according to this aspect, the reference value is acquired by applying the ratio to a CD45RA-negative population among the FOXP3-positive T cell population acquired based on the result of measuring the control sample (S108). In this way, cells to be noises in acquiring the reference value are removed, and therefore the accuracy of the reference value can be further increased.

In this case, the ratio is set so that a proportion of a number of cells having a higher FOXP3 level to a total cell number of the FOXP3-positive T cell population acquired based on the result of measuring the control sample is 62% or more to 72% or less. The ratio is set in such a way, so that the reference value can be appropriately set as described in the following embodiment.

In addition, the ratio may be set so that the ratio of a number of cells having a higher FOXP3 level relative to a number of the cells having a lower FOXP3 level is 1.62 or more to 2.47 or less in the FOXP3-positive T cell population acquired based on the result of measuring the control sample. The ratio is set in such a way, so that the reference value can be appropriately set as described in the following embodiment.

In the effector type regulatory T cell detecting method according to this aspect, when the CD45RA-negative cell population acquired based on the result of measuring the control sample is represented in a histogram (71) with an axis of the FOXP3 level, a cell population having a FOXP3 level equal to or higher than a FOXP3 level at an end point of a peak waveform in a FOXP3-negative side of the histogram (71) is specified as the FOXP3-positive T cell population (S107). In this way, the FOXP3-positive T cell population can be appropriately specified.

In this case, when an approximated curve is applied to the peak waveform in the FOXP3-negative side of the histogram (71), a point at which the approximated curve intersects with the axis in a higher value side of the FOXP3 level is set as the end point. In this way, the end point can be uniformly set.

In the effector type regulatory T cell detecting method according to this aspect, based on the result of measuring the biological sample, a cell population which is CD45RA-negative and has a FOXP3 level higher than the reference value is detected as the effector type regulatory T cells. In this way, the effector type regulatory T cells can be detected with high accuracy.

In this case, based on the result of measuring the biological sample, a single living lymphocyte population is extracted (S201 to S203), a CD3-positive cell population is extracted from the extracted lymphocyte population (S204), a CD4-positive cell population is extracted from the extracted CD3-positive cell population (S205), and a cell population which is CD45RA-negative and has a FOXP3 level higher than the reference value among the extracted CD4-positive cell population is detected as the effector type regulatory T cells (S206).

In the effector type regulatory T cell detecting method according to this aspect, the control sample is a sample containing a peripheral blood of a healthy human, or peripheral blood mononuclear cells purified from a peripheral blood of a healthy human.

A second aspect of the present invention relates to an effector type regulatory T cell analyzing device using flow cytometry. The effector type regulatory T cell analyzing device (120) according to this aspect includes:
a measurement section (200) for measuring a biological sample (102) containing a CD4-positive T cell population; and
a control section (310) in which, based on a result of measuring a control sample (101) containing a CD4-positive T cell population by the measurement section (200), a FOXP3 level being a division reference for dividing a FOXP3-positive T cell population contained in the CD4-positive T cell population at a predetermined ratio is acquired as a reference value to be used for measuring the biological sample (102), and based on a result of measuring the biological sample (102) containing the CD4-positive T cell population by the measurement section (200), effector type regulatory T cells are detected from a cell population having a FOXP3 level higher than the reference value.

The effector type regulatory T cell analyzing device (120) according to this aspect can generate the same effect as in the first aspect.

In the effector type regulatory T cell analyzing device (120) according to this aspect, the measurement section (200) measures the control sample (101) for each measurement of one biological sample (102), and the control section (310) can be configured so that the reference value is applied to detection of the effector type regulatory T cells in analysis of one biological sample (102).

In the effector type regulatory T cell analyzing device (120) according to this aspect, the measurement section (200) measures the control sample (101) for each measurement of a plurality of biological samples (102), and the control section (310) can be configured so that the reference value is applied to detection of the effector type regulatory T cells in the analysis of each biological sample (102).

In the effector type regulatory T cell analyzing device (120) according to this aspect, the control section (310) can be configured so that the reference value is acquired by applying the ratio to an entire FOXP3-positive T cell population acquired based on the result of measuring the control sample (101).

In this case, the control section (310) can be configured so that when the CD4-positive T cell population acquired based on the result of measuring the control sample (101) is represented in a histogram (71) with an axis of the FOXP3 level, a cell population having a FOXP3 level equal to or higher than a FOXP3 level at an end point of a peak waveform in a FOXP3-negative side of the histogram (71) is specified as the FOXP3-positive T cell population.

In addition, the control section (310) can be configured so that when an approximated curve is applied to the peak waveform in the FOXP3-negative side of the histogram (71), a point at which the approximated curve intersects with the axis in a higher value side of the FOXP3 level is set as the end point.

In the effector type regulatory T cell analyzing device (120) according to this aspect, the control section (310) can be configured so that a CD45RA-negative population is extracted from the FOXP3-positive T cell population acquired based on the result of measuring the control sample (101), and the reference value is acquired by applying the ratio to the extracted CD45RA-negative population.

In this case, the control section (310) can be configured so that when the CD45RA-negative cell population acquired based on the result of measuring the control sample (101) is represented in the histogram (71) with an axis of the FOXP3 level, a cell population having a FOXP3 level equal to or higher than a FOXP3 level at an end point of the peak waveform in a FOXP3-negative side of the histogram (71) is specified as the FOXP3-positive T cell population.

In addition, the control section (310) can be configured so that when an approximated curve is applied to the peak waveform in the FOXP3-negative side of the histogram (71), a point at which the approximated curve intersects with the axis in a higher value side of the FOXP3 level is set as the end point.

In the effector type regulatory T cell analyzing device (120) according to this aspect, the control section (310) can be configured so that, based on the result of measuring the biological sample (102), a cell population which is CD45RA-negative and has a FOXP3 level higher than the reference value is detected as the effector type regulatory T cells.

In this case, the control section (310) can be configured so that, based on the result of measuring the biological sample (102), a single living lymphocyte population is extracted, a CD3-positive cell population is extracted from the extracted lymphocyte population, a CD4-positive cell population is extracted from the extracted CD3-positive cell population, and a cell population which is CD45RA-negative and has a FOXP3 level higher than the reference value among the extracted CD4-positive cell population is detected as the effector type regulatory T cells.

A third aspect of the present invention relates to an effector type regulatory T cell analyzing system. An effector type regulatory T cell analyzing system (100) according to this aspect includes the analyzing device (120) according to the second aspect, and a labeling device (110) for fluorescently labeling test substances in the control sample (101) and the biological sample (102).

The effector type regulatory T cell analyzing system according to this aspect can generate the same effect as in the first and second aspects, and allows automization of the fluorescent labeling for the test substances in the control sample and the biological sample. Thereby, a plurality of samples can be prepared under uniform conditions.

Also disclosed is a program for computer of a flow cytometer for analyzing an effector type regulatory T cell with the functions of:
acquiring, based on a result of measuring a control sample (101) containing a CD4-positive T cell population, a FOXP3 level being a division reference for dividing a FOXP3-positive T cell population contained in the CD4-positive T cell population at a predetermined ratio, as a reference value to be used for measuring a biological sample (102); and
detecting, based on a result of measuring the biological sample (102) containing a CD4-positive T cell population, effector type regulatory T cells from a cell population having a FOXP3 level higher than the reference value.

### Advantageous Effects of Invention

According to the present invention, the effector type regulatory T cells can be stably detected with high accuracy.

### Brief Description of Drawings

FIG. 1(a) is a flowchart presenting a processing for acquiring a reference value by measuring a control sample according to Embodiment 1. FIG. 1(b) is a schematic diagram presenting a histogram represented in the processing for acquiring the reference value according to Embodiment 1. FIG. 1(c) is a flowchart presenting a processing for detecting effector type regulatory T cells contained in biological samples according to Embodiment 1.
FIG. 2 is a detail flowchart presenting the processing for acquiring the reference value according to Embodiment 1.
FIG. 3(a) is a diagram illustrating a scattergram including all particles according to Embodiment 1. FIG. 3(b) is a diagram illustrating a scattergram including extracted single cells according to Embodiment 1.
FIG. 3(c) is a diagram illustrating a scattergram including extracted living cells according to Embodiment 1.
FIG. 3(d) is a diagram illustrating a scattergram including extracted lymphocytes according to Embodiment 1.
FIG. 3(e) is a diagram illustrating a scattergram including extracted CD3-positive T cells according to Embodiment 1.
FIG. 3(f) is a diagram illustrating a scattergram including extracted CD4-positive T cells according to Embodiment 1.
FIG. 4 is a diagram illustrating a histogram acquired by representing the extracted CD4-positive T cells according to Embodiment 1 with an axis of a FOXP3 level.
FIG. 5 is a detail flowchart presenting the processing for detecting the effector type regulatory T cells according to Embodiment 1.
FIG. 6(a) is a scattergram prepared in a case that the biological sample according to Embodiment 1 is a lymphocyte which infiltrates gastric cancer tissues of a patient with gastric cancer.
FIG. 6(b) is a schematic diagram of a histogram acquired by representing the extracted CD4-positive T cells according to Embodiment 1 with an axis of a CD45RA level.
FIG. 6(c) is a histogram acquired by representing the extracted CD4-positive T cells with an axis of the FOXP3 level in a case that the biological sample according to Embodiment 1 is a lymphocyte which infiltrates gastric cancer tissues of a patient with gastric cancer.
FIG. 7(a) is a diagram presenting a boundary value visually set in a scattergram in a case that the biological sample according to verification of Embodiment 1 is a lymphocyte which infiltrates gastric cancer tissues of a patient with gastric cancer.
FIG. 7(b) is a scattergram in which a boundary value is set in a case that the biological sample according to verification of Embodiment 1 is a peripheral blood mononuclear cell of a healthy human.
FIG. 7(c) is a histogram in which a boundary value is set in a case that the biological sample according to verification of Embodiment 1 is a peripheral blood mononuclear cell of a healthy human.
FIG. 8(a) is a histogram in which a boundary value is set in a case that the biological sample according to verification of Embodiment 1 is a peripheral blood mononuclear cell of a healthy human.
FIG. 8(b) is a histogram in which a boundary value is set in a case that the biological sample according to verification of Embodiment 1 is a peripheral blood mononuclear cell of a healthy human.
FIG. 8(c) is a histogram in which a boundary value is set in a case that the biological sample according to verification of Embodiment 1 is a peripheral blood mononuclear cell of a healthy human.
FIG. 9(a) is a diagram presenting a state that a ratio is applied to a histogram acquired based on the control sample according to verification of Embodiment 1.
FIG. 9(b) is a scattergram in which a reference value is set in a case that the biological sample according to verification of Embodiment 1 is a lymphocyte which infiltrates gastric cancer tissues of a patient with gastric cancer.
FIG. 10(a) is a scattergram for explaining manual fractionation in a comparative example according to verification of Embodiment 1.
FIG. 10(b) is a scattergram for explaining manual fractionation in the comparative example according to verification of Embodiment 1.
FIG. 10(c) is a scattergram for explaining manual fractionation in the comparative example according to verification of Embodiment 1.
FIG. 10(d) is a scattergram for explaining manual fractionation in the comparative example according to verification of Embodiment 1.
FIG. 10(e) is a scattergram for explaining manual fractionation in the comparative example according to verification of Embodiment 1.
FIG. 10(f) is a scattergram for explaining manual fractionation in the comparative example according to verification of Embodiment 1.
FIG. 11 is a detail flowchart presenting a processing for acquiring a reference value according to Embodiment 2.
FIG. 12(a) is a scattergram containing CD4-positive T cells extracted based on a control sample according to Embodiment 2.
FIG. 12(b) is a histogram acquired by representing the CD4-positive T cells extracted based on the control sample according to Embodiment 2 with an axis of the CD45RA level.
FIG. 12(c) is a contour diagram in a scattergram containing the CD4-positive T cells extracted based on the control sample according to Embodiment 2.
FIG. 13(a) is a diagram presenting a reference value and a fractionation value set in a scattergram in a case that a biological sample according to Embodiment 2 is a lymphocyte which infiltrates gastric cancer tissues of a patient with gastric cancer.
FIG. 13(b) is a histogram acquired by representing the extracted CD4-positive T cells with an axis of the FOXP3 level in a case that the biological sample according to Embodiment 2 is a lymphocyte which infiltrates gastric cancer tissues of a patient with gastric cancer
FIG. 14(a) is a histogram in which a boundary value is set in a case that the biological sample according to verification of Embodiment 2 is a peripheral blood mononuclear cell of a healthy human.
FIG. 14(b) is a histogram in which a boundary value is set in a case that the biological sample according to verification of Embodiment 2 is a peripheral blood mononuclear cell of a healthy human.
FIG. 14(c) is a histogram in which a boundary value is set in a case that the biological sample according to verification of Embodiment 2 is a peripheral blood mononuclear cell of a healthy human.
FIG. 15(a) is a diagram presenting a state that a ratio is applied to a histogram acquired based on the control sample according to verification of Embodiment 2.
FIG. 15(b) is a scattergram in which a reference value is set in a case that the biological sample according to verification of Embodiment 2 is a lymphocyte which infiltrates gastric cancer tissues of a patient with gastric cancer.
FIG. 16(a) is a detail flowchart presenting a processing for detecting effector type regulatory T cells according to Embodiment 3.
FIG. 16(b) is a diagram presenting a region of the effector type regulatory T cells, a region of naive type regulatory T cells, and a region of inactive type regulatory T cells in a scattergram according to Embodiment 3.
FIG. 17 is a block diagram presenting a configuration of an analyzing system for carrying out the analyses according to Embodiments 1 to 3.
FIG. 18 is a flow chart presenting a processing executed in the analyzing system for carrying out the analyses according to Embodiments 1 to 3.
FIG. 19 is a flow chart presenting a processing executed in the analyzing system for carrying out the analyses according to Embodiments 1 to 3.
FIG. 20 is a diagram presenting a configuration of a screen displayed on a display section in the analyzing system for carrying out the analyses according to Embodiments 1 to 3.
FIG. 21 is a schematic diagram for explaining the related technology.

### Description of Embodiments

In the following embodiments, a control sample is measured to acquire a reference value, and effector type regulatory T cells contained in a biological sample are detected using the acquired reference value.

The control sample refers to a sample measured for acquiring a reference value used for measuring the biological sample, and includes e.g. a peripheral blood mononuclear cell population equivalent to that of a healthy human. The control sample includes a wide range of samples or the like containing a peripheral blood itself of a healthy human, peripheral blood mononuclear cells purified from a peripheral blood of a healthy human by a density gradient method, or the like. Note that the peripheral blood mononuclear cells (PBMC) include monocytes and lymphocytes. The healthy human refers to a person who does not suffer from a certain chronic disease and has no difficulty in daily life. The biological sample refers to a sample including peripheral blood mononuclear cells of a subject, e.g. a sample containing lymphocytes (TIL) which infiltrate cancer tissues of a subject.

Additionally, in the following embodiments, for convenience sake, explanation is made on the premise that scattergrams and histograms are actually prepared, but the scattergrams and the histograms are not necessarily prepared. For example, instead of the processing based on the prepared scattergrams and histograms, an equivalent procedure may be executed by data processing.

### <Embodiment 1>

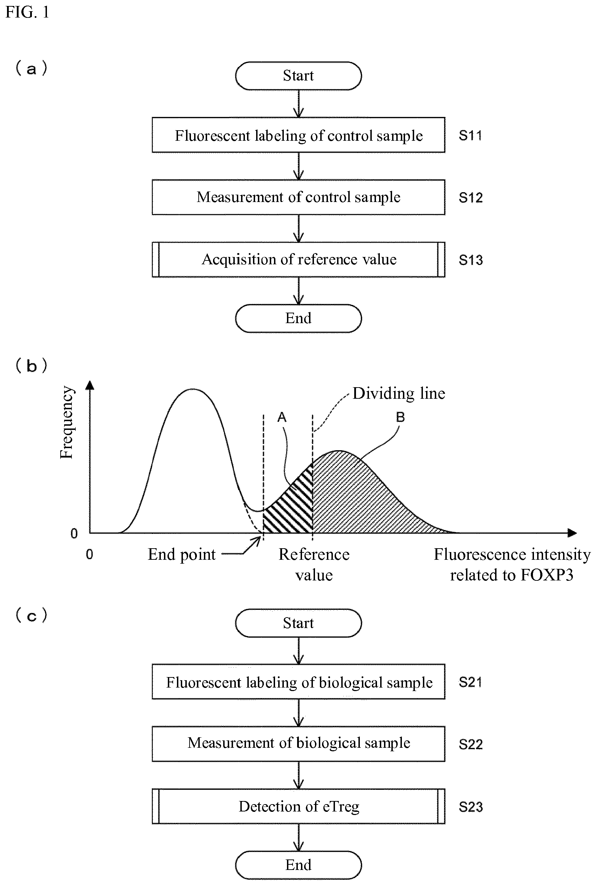

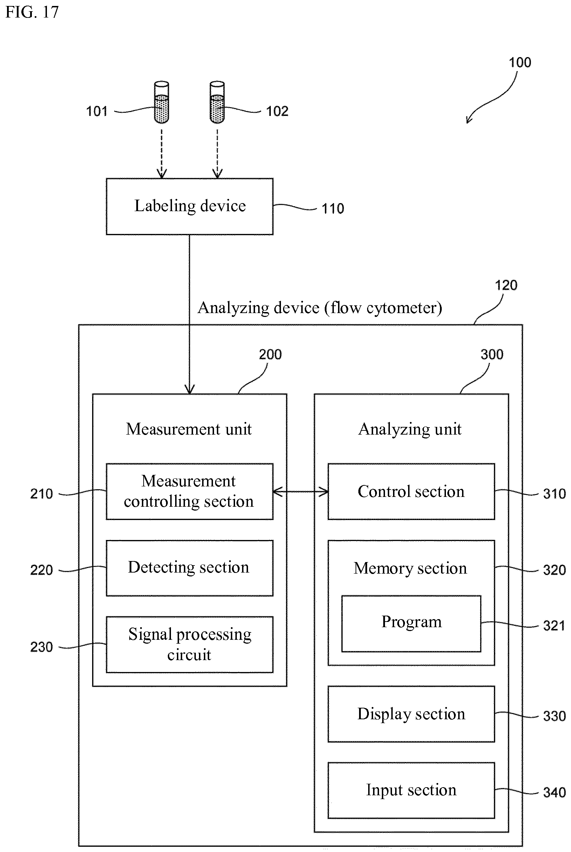

As presented in FIG. 1(a), the method for acquiring the reference value by measuring the control sample includes steps S11 to S13. Explanation of FIG. 1(a) will be explained with reference to the graph presented in FIG. 1(b) as appropriate. As presented in FIG. 1(c), the method for detecting the effector type regulatory T cells (eTreg) contained in the biological samples using the reference value acquired by the method in FIG. 1(a) includes steps S21 to S23 Hereinafter, a case that the operator executes the methods in FIG. 1(a) and FIG. 1(c) using a device will be explained. Incidentally, each step in FIG. 1(a) and FIG. 1(c) may be automatically executed by the device. The configuration of the device for executing each step in FIG. 1(a) and FIG. 1(c) will be explained with reference to FIG. 17.

The processing for measuring the control sample to acquire a reference value will be explained with reference to FIG. 1(a).

In step S11, the operator fluorescently labels the test substance in the control sample. Specifically, a fluorescent dye which stains dead cells more strongly than living cells, a fluorescent dye for staining a surface antigen CD3, a fluorescent dye for staining a surface antigen CD4, a fluorescent dye for staining a surface antigen CD8, a fluorescent dye for staining a surface antigen CD45RA, and a fluorescent dye for staining a transcription factor FOXP3 of the regulatory T cell (Treg) fluorescently label each corresponding test substance in cells contained in the control sample.

The test substances are fluorescently stained by binding each labeling antibody having each fluorescent dye to the test substance. For example, as a labeling antibody having the fluorescent dye which stains dead cells more strongly than living cells, Fixable Viability Dye (FVD-eFluor (registered trademark) 506) (affymetrix eBioscence) is used. As a labeling antibody having the fluorescent dye for staining the surface antigen CD3, anti-human CD3 Alexa Fluor (registered trademark) 700 (affymetrix eBioscence) is used. As a labeling antibody having the fluorescent dye for staining the surface antigen CD4, anti-human CD4 APC-eFluor (registered trademark) 780 (affymetrix eBioscence) is used. As a labeling antibody having the fluorescent dye for staining the surface antigen CD8, anti-human CD8a PE-Cy7 (affymetrix eBioscence) is used. As a labeling antibody having the fluorescent dye for staining the surface antigen CD45RA, anti-human CD45RA FITC (affymetrix eBioscence) is used. As a labeling antibody having the fluorescent dye for staining the FOXP3, anti-human FoxP3 PE (affymetrix eBioscence) is used.

In step S12, the operator measures the fluorescently labeled control sample. In step S12, signals attributed to forward-scattered lights, side-scattered lights, and fluorescences generated from the control sample are acquired based on the flow cytometry.

Specifically, the operator supplies the control sample fluorescently labeled in step S11 to a detecting section of an analyzing device (flow cytometer). The detecting section causes the supplied control sample to flow into a flow cell. The detecting section emits light to the control sample flowing through the flow cell. Thereby, particles such as cells contained in the control sample are irradiated with light, the forward-scattered lights and the side-scattered lights are generated from the particles, and fluorescences having different wavelengths are generated from each fluorescent dye. The detecting section outputs signals attributed to the forward-scattered lights, the side-scattered lights, and the fluorescences. Then the signals output from the detecting section are processed into waveform signals for each particle to acquire feature parameters such as a peak value, an area, and a width of the waveform from the waveform signals, and the acquired feature parameters are stored as a measurement result in a memory section in the analyzing device.

In step S13, based on the result of measuring the control sample in step S12, the operator acquires a FOXP3 level which is a division reference for dividing the FOXP3-positive T cell population contained in the CD4-positive T cell population at a predetermined ratio, as a reference value used for measuring the biological sample.

Specifically, in the analyzing device, the CD4-positive T cells are extracted based on the signals acquired in step S12, and the extracted CD4-positive T cells are represented in the histogram with the axis of the FOXP3 level as presented in FIG. 1(b). In this histogram, the abscissa axis represents the FOXP3 level, and the ordinate axis represents a frequency. The FOXP3 level refers to a value indicating what degree of the FOXP3 is contained in the cell, specifically an intensity of the fluorescence generated from the fluorescent dye for staining the FOXP3. In the analyzing device, the cell population having the FOXP3 level equal to or higher than that of the end point in the higher value side of the peak waveform in the FOXP3-negative side of the histogram is specified as the FOXP3-positive T cell population.

Subsequently, in the analyzing device, the specified FOXP3-positive T cell population is divided at a ratio previously stored in the memory section. That means, as presented in FIG. 1(b), under a condition that numbers of FOXP3-positive T cells contained in the left side and right side of a dividing line are defined A and B respectively, the dividing line is set so that a ratio A:B is the ratio previously stored in the memory section. Then, in the analyzing device, the FOXP3 level which is the division reference for dividing the FOXP3-positive T cell population at a predetermined ratio is acquired as a reference value, and stored in the memory section.

Incidentally A and B are not limited to the numbers of cells, and may be areas of the lower portion of the waveform portion at the left side and the right side respectively of the dividing line, on the waveform portion indicating the FOXP3-positive T cell population as presented in FIG. 1(b). In this case, the dividing line is set so that the area ratio A:B of the FOXP3-positive T cell population contained in the left side and right side of the dividing line is a predetermined ratio.

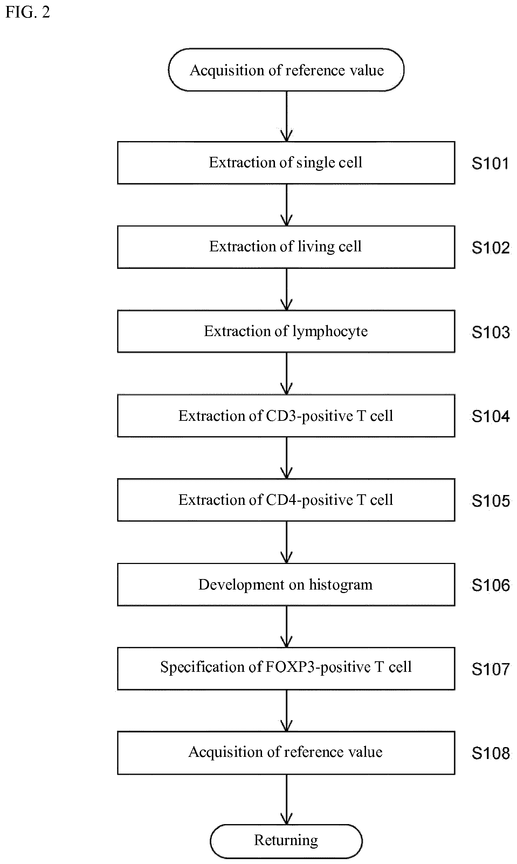

Thus, the reference value is acquired by measuring the control sample. Such reference value is acquired before detecting effector type regulatory T cells from a biological sample collected from a subject. The process for acquiring the reference value in step S13 will be explained in detail with reference to FIG. 2 later.

The processing for detecting the effector type regulatory T cells contained in the biological sample using the acquired reference value will be explained with reference to FIG. 1(c).

In step S21, the operator fluorescently labels the test substance in the biological sample. In step S21, the test substance in cells contained in the biological sample is fluorescently labeled with the same fluorescent dye as in step S11 in FIG. 1(a). In step S22, the operator measures the fluorescently labeled biological sample. In the same manner as in step S12 in FIG. 1(a), in step S22, signals attributed to forward-scattered lights, side-scattered lights, and fluorescences generated from the biological sample are acquired by the detecting section of the analyzing device (flow cytometer). Then the signals output from the detecting section are processed into waveform signals for each particle to acquire feature parameters such as a peak value, an area, and a width of the waveform from the waveform signals, and the acquired feature parameters are stored as a measurement result in a memory section in the analyzing device.

In step S23, on the basis of the result of measuring the biological sample in step S22, the operator detects the effector type regulatory T cells based on the cell population having a FOXP3 level higher than the reference value.

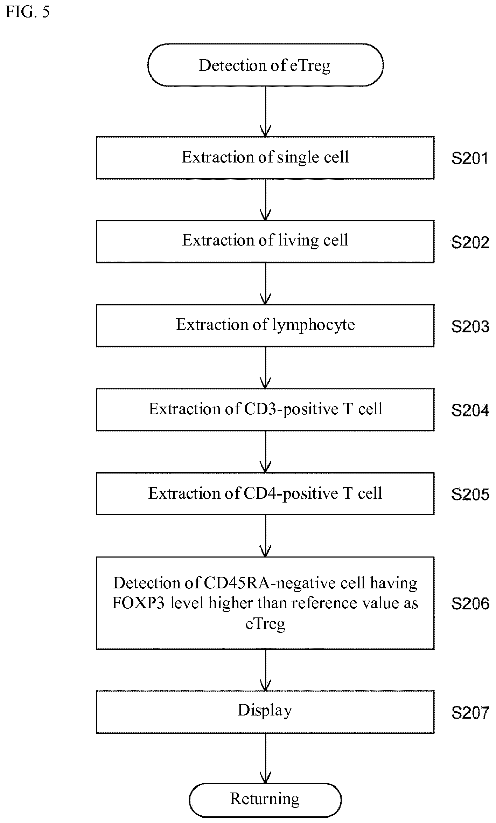

Specifically, in the analyzing device, the CD4-positive T cells are extracted based on the signals acquired in step S22. In the analyzing device, the reference value acquired in step S13 in FIG. 1(a) is read out from the memory section, and the cells having a FOXP3 level higher than the reference value are extracted from the extracted CD4-positive T cells. In the analyzing device, among the extracted cells having a FOXP3 level higher than the reference value, the CD45RA-negative cells are detected as the effector type regulatory T cells. The process for detecting the effector type regulatory T cells in step S23 will be explained in detail with reference to FIG. 5 later. Incidentally, in the extracted cells having a FOXP3 level higher than the reference value, the CD45RA-positive cell level is generally low, and therefore the cells having a FOXP3 level higher than the reference value may be detected as the effector type regulatory T cells without extraction according to the CD45RA level.

As described above, the reference value can be uniformly set, because the reference value is set by dividing the FOXP3-positive T cell population acquired by measuring the control sample at a predetermined ratio. In addition, the reference value can be appropriately set even if the measurement environment changes by using a sample containing a peripheral blood mononuclear cell population equivalent to that of a healthy human as a control sample. That means, an appropriate reference value can be set according to Embodiment 1, even with a change in the measurement environment such as replacement of a reagent, deterioration of the reagent, change in an environmental temperature, and change in a preparation quantity of the reagent or the sample. Furthermore, since the reference value has been acquired by previously measuring the control sample, a reference value corresponding to the measurement environment can be set as a reference value for measuring the biological sample. As described above, since the reference value used for measuring the biological sample can be uniformly and appropriately set, the effector type regulatory T cells can be stably detected with high accuracy.

Incidentally, the reference value-acquiring processing presented in FIG. 1(a) may be executed for each measurement of one biological sample, or for each measurement of a plurality of biological samples.

That means, the control sample is measured for each measurement of one biological sample to acquire a reference value, and the acquired reference value may be used for detecting the effector type regulatory T cells in measurement of one biological sample. In this case, the reference value is set for each measurement of the biological sample, and therefore a reference value adapted to the measurement environment for measuring the biological sample can be set to improve a detection accuracy of the effector type regulatory T cells.

In addition, the control sample is measured for each measurement of the plurality of biological samples to acquire a reference value, and the acquired reference value may be used for detecting the effector type regulatory T cells in the measurement of each biological sample. In this case, a measurement frequency for the control sample can be reduced, and therefore a measurement efficiency for the biological samples can be enhanced. When the plurality of biological samples can be prepared and measured under uniform conditions, this method is particularly effective, like a case that the biological samples are automatically prepared and measured by a device. In addition, since a state of a reagent may be changed at a timing of changing a reagent lot, it is preferable to set a reference value with the control sample again, even when the biological samples are automatically prepared and measured by the device.

The processing for acquiring the reference value will be explained in detail with reference to FIG. 2. In each step described below, processing is executed based on the control sample-measuring result acquired in step S12 in FIG. 1(a).

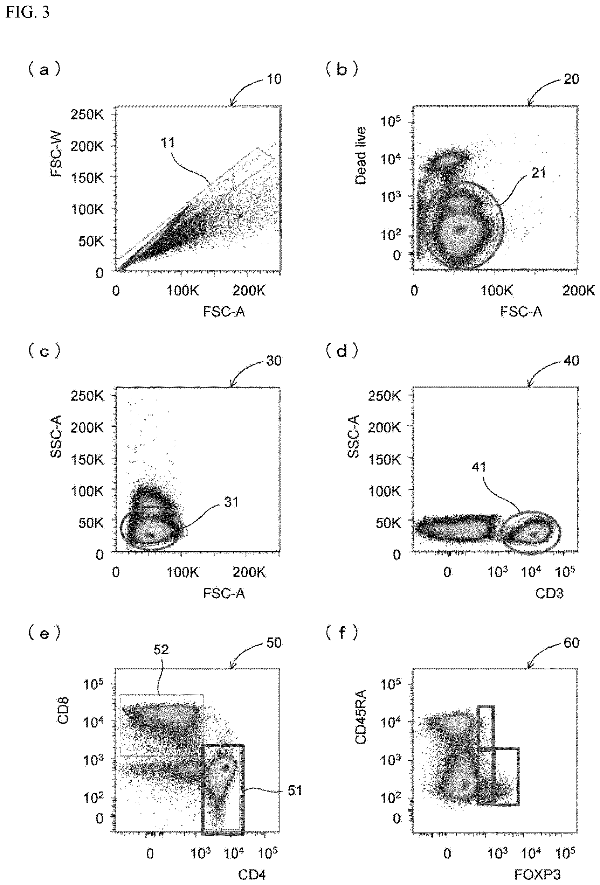

In step S101, in the analyzing device, all particles are plotted on a scattergram 10 based on the measurement result as presented in FIG. 3(a), and particles included in a region 11 are extracted as single cells. On the scattergram 10, the abscissa axis represents an area of a signal waveform attributed to forward-scattered lights, and the ordinate axis represents a width of a signal waveform attributed to forward-scattered lights.

In step S102, in the analyzing device, the single cells extracted in step S101 are plotted on a scattergram 20 as presented in FIG. 3(b), and particles included in a region 21 are extracted as living cells. On the scattergram 20, the abscissa axis represents an area of a signal waveform attributed to forward-scattered lights, and the ordinate axis represents a peak value of a signal waveform attributed to fluorescences generated from the fluorescent dye which stains dead cells more strongly than living cells.

In step S103, in the analyzing device, the living cells extracted in step S102 are plotted on a scattergram 30 as presented in FIG. 3(c), and particles included in a region 31 are extracted as lymphocytes. On the scattergram 30, the abscissa axis represents an area of a signal waveform attributed to forward-scattered lights, and the ordinate axis represents an area of a signal waveform attributed to side-scattered lights.

Note that the order for executing steps S101 to S103 is not limited to the order presented in FIG. 2. Each of steps S101 to S103 may be executed in any order.

In step S104, in the analyzing device, the lymphocytes extracted in step S103 are plotted on a scattergram 40 as presented in FIG. 3(d), and particles included in a region 41 are extracted as CD3-positive T cells. On the scattergram 40, the abscissa axis represents a peak value of a signal waveform attributed to the fluorescences generated from the fluorescent dye for staining the surface antigen CD3, and the ordinate axis represents an area of a signal waveform attributed to the side-scattered lights.

In step S105, in the analyzing device, the CD3-positive T cells extracted in step S104 are plotted on a scattergram 50 as presented in FIG. 3(e), and particles included in a region 51 are extracted as CD4-positive T cells. On the scattergram 50, the abscissa axis represents a peak value of a signal waveform attributed to the fluorescences generated from the fluorescent dye for staining the surface antigen CD4, and the ordinate axis represents a peak value of a signal waveform attributed to the fluorescences generated from the fluorescent dye for staining the surface antigen CD8. Incidentally, the region 52 refers to a region including the CD8-positive T cells.

When the CD4-positive T cells extracted in step S105 are plotted on a scattergram 60, particles are distributed e.g. as presented in FIG. 3(f). On the scattergram 60, the abscissa axis represents a peak value of a signal waveform attributed to the fluorescences generated from the fluorescent dye for staining the FOXP3, and the ordinate axis represents a peak value of a signal waveform attributed to the fluorescences generated from the fluorescent dye for staining the surface antigen CD45RA.

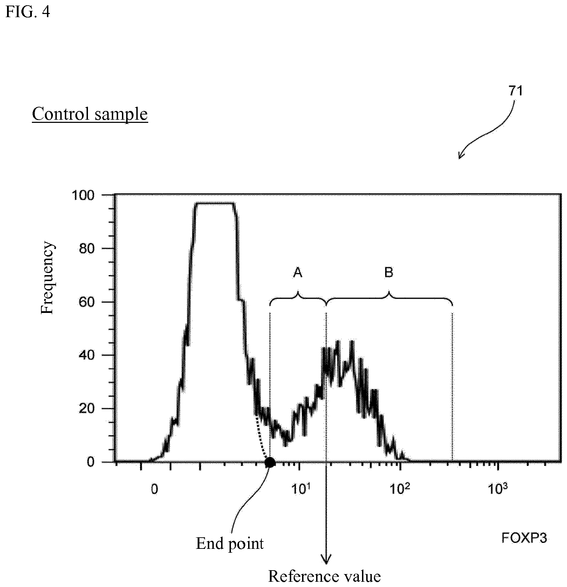

In step S106, in the analyzing device, the CD4-positive T cells extracted in step S105 are represented in a histogram 71 as presented in FIG. 4. On the histogram 71, the abscissa axis represents a peak value of a signal waveform attributed to the fluorescences generated from the fluorescent dye for staining the FOXP3, and the ordinate axis represents a frequency. The abscissa axis of the histogram 71 is the same as the abscissa axis of the scattergram 60 in FIG. 3(f).

On the histogram 71 presented in FIG. 4, a frequency exceeding about 100 is omitted for convenience sake. Also on the histograms 71 and 72 presented in the subsequent figures, frequencies exceeding a predetermined value will be similarly omitted as appropriate.

In step S107, in the analyzing device, the FOXP3-positive T cells are specified based on the histogram 71 acquired by representation in step S106.

Specifically, a cell population having the FOXP3 level equal to or higher than that of the end point in the higher value side of the peak waveform in the FOXP3-negative side of the histogram 71 is specified as the FOXP3-positive T cells. Thereby, the FOXP3-positive T cell population can be appropriately specified.

In this case, when an approximated curve is applied to the peak waveform in the FOXP3-negative side of the histogram (71) i.e. the mountain-like waveform on the left side, this end point is set to a point at which the approximated curve intersects with the axis of the FOXP3 level in the higher value side of the FOXP3 level. More specifically, an approximated curve for a normal distribution of the mountain-like waveform on the left side is acquired by Gaussian fitting, and a point at which the approximated curve intersects with the axis of the FOXP3 level in the higher value side is set as the end point. In this way, the end point can be uniformly set. In FIG. 4, the approximated curve near the end point is represented by a thick dotted line.

In step S108, in the analyzing device, a predetermined ratio is applied to the entire FOXP3-positive T cell population specified in step S107, and a FOXP3 level which is a division reference for dividing the FOXP3-positive T cell population at this ratio is acquired as a reference value. The predetermined ratio is previously stored in the memory section, and is read out from the memory section in each measurement of the biological sample.

Herein, as explained with reference to FIG. 1(b), the numbers of FOXP3-positive T cells included on the left and right sides of the dividing line are defined as A and B respectively. A is a number of cells having the lower FOXP3 level, and B is a number of cells having the higher FOXP3 level. In the analyzing device, a dividing line is set so that A : B is a ratio previously stored in the memory section, and the FOXP3 level at which the dividing line intersects with the abscissa axis is acquired as a reference value.

In Embodiment 1, the ratio is set so that a ratio B/A of the number B of the cells having the higher FOXP3 level to the number A of the cells having the lower FOXP3 level is 1.35 or more to 2.48 or less. This ratio is a communis ratio found by the inventors of the present invention, as will be described later. That means, the inventors have found that, based on this ratio, the reference value can be appropriately set regardless of the measurement environment and the like, and the effector type regulatory T cells can be detected with high accuracy. The reason why the inventors have determined that this ratio is communis will be explained later in verification of Embodiment 1. In Embodiment 1, the ratio B/A is set to 1.96.

Incidentally, the ratio may be set so that a proportion B/(A + B) of the number B of the cells having the higher FOXP3 level to the total cell number (A + B) of the FOXP3-positive T cell population is 57% or more to 72% or less. In this case, a range of the B/A is substantially the same as in the case of the B/A set to 1.35 or more to 2.48 or less, and therefore the reference value can be appropriately set also in this case.

The processing for detecting the effector type regulatory T cells will be explained in detail with reference to FIG. 5. In each step described below, processing is executed based on the biological sample-measuring result acquired in step S22 in FIG. 1(c).

In step S201, in the analyzing device, all particles are plotted on the scattergram 10 based on the measurement result in the same manner as in step S101 in FIG. 2, and the particles included in the region 11 are extracted as single cells. In step S202, in the analyzing device, the single cells extracted in step S201 are plotted on the scattergram 20 in the same manner as in step S102 in FIG. 2, and the particles included in the region 21 are extracted as living cells. In step S203, in the analyzing device, the living cells extracted in step S202 are plotted on the scattergram 30 in the same manner as in step S103 in FIG. 2, and the particles included in the region 31 are extracted as lymphocytes.

Note that the order for executing steps S201 to S203 is not limited to the order presented in FIG. 5. Each of steps S201 to S203 may be executed in any order.

In step S204, in the analyzing device, the lymphocytes extracted in step S203 are plotted on the scattergram 40 in the same manner as in step S104 in FIG. 2, and the particles included in the region 41 are extracted as CD3-positive T cells. In step S205, in the analyzing device, the CD3-positive T cells extracted in step S204 are plotted on the scattergram 50 in the same manner as in step S105 in FIG. 2, and the particles included in the region 51 are extracted as CD4-positive T cells.

In step S206, in the analyzing device, cells which are CD45RA-negative and have a FOXP3 level higher than the reference value are detected as effector type regulatory T cells.

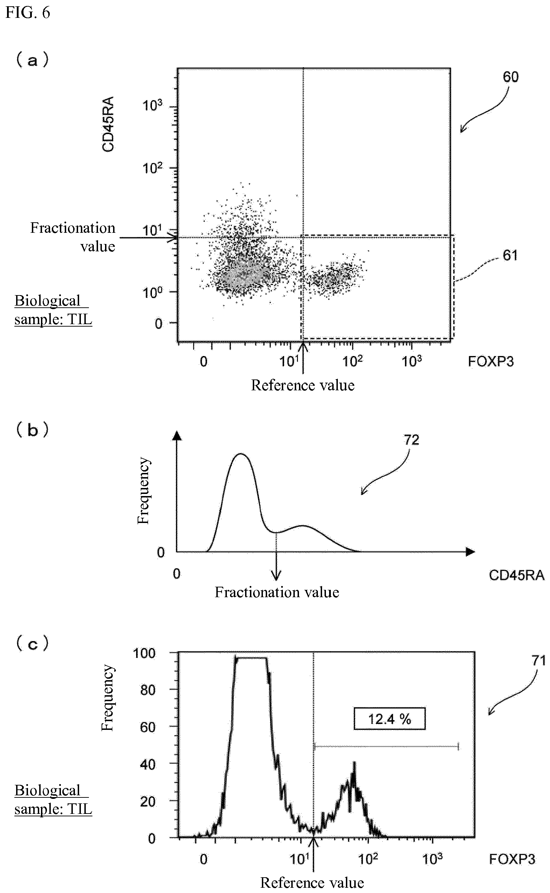

Specifically, in the analyzing device, the CD4-positive T cells extracted in step S205 are represented in a scattergram 60 as presented in FIG. 6(a). The axes of the scattergram 60 are set in the same manner as on the scattergram 60 presented in FIG. 3(f). That means, the abscissa axis represents a peak value of a signal waveform attributed to the fluorescences generated from the fluorescent dye for staining the FOXP3, and the ordinate axis represents a peak value of a signal waveform attributed to the fluorescences generated from the fluorescent dye for staining the surface antigen CD45RA. Incidentally, the scattergram 60 in FIG. 6(a) is prepared in a case that the biological sample is a lymphocyte (TIL) which infiltrate gastric cancer tissues of a patient with gastric cancer.

In the analyzing device, a vertically extending straight line with an abscissa axis value as a reference value, and a horizontally extending straight line with an ordinate axis value as the fractionation value are set on the scattergram 60. The reference value used in this scattergram is the reference value acquired in step S108 in FIG. 2. The fractionation value is acquired based on the histogram 72 presented in FIG. 6(b).

The histogram 72 in FIG. 6(b) is a schematic diagram of a histogram acquired by representing all cells on the scattergram 60 in FIG. 6(a). On the histogram 72, the abscissa axis represents a peak value of a signal waveform attributed to the fluorescences generated from the fluorescent dye for staining the surface antigen CD45RA, and the ordinate axis represents a frequency. The abscissa axis of the histogram 72 corresponds to the ordinate axis of the scattergram 60. In the analyzing device, the CD45RA level at which the frequency is minimum between the peak waveform at the CD45RA-negative side and the peak waveform at the CD45RA-positive side is set as a fractionation value. Cells having a CD45RA level lower than the fractionation value are CD45RA-negative cells, and cells having a CD45RA level higher than the fractionation value are CD45RA-positive cells.

On the scattergram 60 presented in FIG. 6(a), the region 61 is a region where the CD45RA level is higher than the reference value acquired in step S108 in FIG. 2 and lower than the fractionation value acquired based on the histogram 72 in FIG. 6(b). In step S206, in the analyzing device, the cells included in the region 61 on the scattergram 60 are detected as the effector type regulatory T cells. In such a way, when the cells in the region 61 are detected as the effector type regulatory T cells, the effector type regulatory T cells can be detected with high accuracy.

On the histogram 71 in FIG. 6(c), all cells distributed on the scattergram 60 in FIG. 6(a) are represented. The axes of the histogram 71 in FIG. 6(c) are set in the same manner as in the histogram 71 presented in FIG. 4. In the examples presented in FIG. 6(a) and FIG. 6(c), a proportion of the cells having a FOXP3 level higher than the reference value to the entire CD4-positive T cells is 12.4%. FIG. 6(a) and FIG. 6(c) indicate that the cells in the region 61 can be accurately detected as the effector type regulatory T cells even when the biological sample is a lymphocyte (TIL) which infiltrates gastric cancer tissues.

Incidentally, a number of the CD45RA-positive cells immediately above the region 61 is small, as presented in FIG. 6(a). Thus, in the analyzing device, among the CD4-positive T cells, cells having a FOXP3 level higher than the reference value may be detected as the effector type regulatory T cells in step S206. In this case, in measuring the biological samples, measurement of fluorescences generated from the fluorescent dye for staining the surface antigen CD45RA can be omitted.

In step S207, in the analyzing device, information about the effector type regulatory T cells detected in step S206 is displayed on the display section. In step S207, e.g. the number of the effector type regulatory T cells, the proportion of the effector type regulatory T cells to the entire CD4-positive T cells, the proportion of the effector type regulatory T cells to the cells having a FOXP3 level higher than the reference value, and the like are displayed.

### <Verification of Embodiment 1>

Next, a circumstance that the inventors have found the communis ratio B/A regardless of the measurement environment will be explained.

In verification of the inventors described below, samples based on the lymphocytes (TIL) which infiltrate gastric cancer tissues of patients with gastric cancer, and samples based on peripheral blood mononuclear cells of healthy humans were prepared in the following procedure.

In verification of the inventors, first, a staining reaction was carried out with FVD (Fixable Viability Dye) for 10 to 15 minutes to remove dead cells. Next, in verification of the inventors, to minimize a non-specific binding of an antibody to be subsequently added without washing, Fc block (BD Fc Block (registered trademark) Reagent for human) (BD Bioscience) is added to the reactant, which was reacted at 4°C for 10 to 20 minutes. After that, in verification of the inventors, to stain the antibodies on the cell surfaces, anti-human CD3, CD4, CD8a and CD45RA antibodies were added to the reactant, which was further subjected to an antibody reaction for 30 minutes. These antibodies are anti-human CD3 Alexa Fluor (registered trademark) 700 (affymetrix eBioscence), anti-human CD4 APC-eFluor (registered trademark) 780 (affymetrix eBioscence), anti-human CD8a PE-Cy7 (affymetrix eBioscence), and anti-human CD45RA FITC (affymetrix eBioscence) respectively.

Subsequently, in verification of the inventors, the cells were washed with PBS (-) containing 2% PBS, and then subjected to cell immobilization and membrane permeation treatment using FoxP3/Transcription Factor Staining Buffer Set (affymetrix eBioscience) for 30 to 60 minutes to stain the inside of the cells. In verification of the inventors, the cells were further washed, and then an anti-FoxP3 antibody reaction was carried out for 30 to 60 minutes to stain the inside of the cells. In verification of the inventors, the cells were washed, and then multicolor measurement was carried out using a flow cytometer "CyFlow space" (manufactured by Sysmex Corporation).

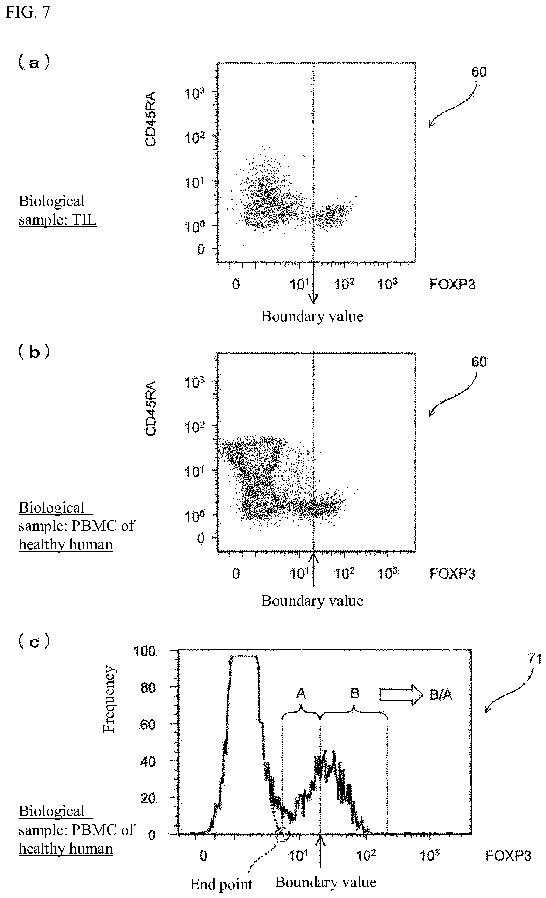

In verification of the inventors, samples based on the lymphocytes (TIL) which infiltrate gastric cancer tissues of patients with gastric cancer were measured, and the CD4-positive T cells were extracted in the same procedure as in steps S201 to S205 in FIG. 5. FIG. 7(a) is a diagram presenting the scattergram 60 acquired by representing the CD4-positive T cells extracted from TIL. In verification of the inventors, a boundary value for distinguishing the effector type regulatory T cells from other cells was visually acquired on the scattergram 60 presented in FIG. 7(a). At this time, in verification of the inventors, the boundary value was acquired using a TIL capable of easily distinguishing the effector type regulatory T cells from other cells as illustrated in FIG. 7(a).

Subsequently, in verification of the inventors, samples based on peripheral blood mononuclear cells of healthy humans were measured in the same batch, and the CD4-positive T cells were extracted and the histogram 71 was prepared in the same procedure as in steps S201 to S205 in FIG. 5. Incidentally, when a plurality of measurements are carried out in the same batch, the environments in each measurement are substantially the same. In the same batch, e.g. reagent lots, situations of reagent deterioration, environmental temperatures, preparation quantities of reagents and samples, and the like are substantially equal to each other in each measurement.

FIG. 7(b) and FIG. 7(c) are diagrams presenting the scattergram 60 and the histogram 71 respectively acquired by representing the CD4-positive T cells extracted from peripheral blood mononuclear cells of healthy humans. FIG. 7(b) and FIG. 7(c) present states where the boundary value acquired in FIG. 7(a) is applied.

In FIG. 7(c), in verification of the inventors, an end point was set based on the peak waveform in the FOXP3-negative side in the same procedure as explained with reference to FIG. 4. Then, in verification of the inventors, the number of the cells between the end point and the boundary value was defined as A, and the number of the cells having a FOXP3 level higher than the boundary value was defined as B to acquire a ratio B/A.

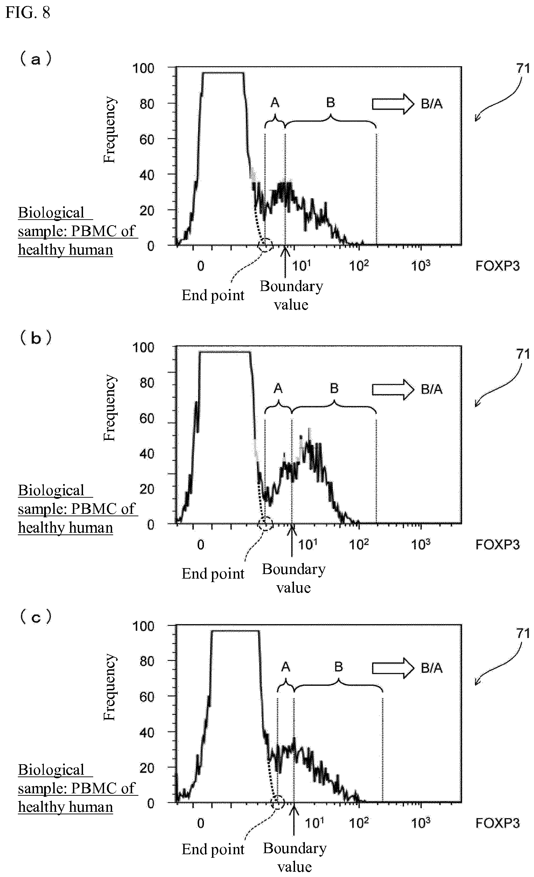

In verification of the inventors, the procedure of acquiring the ratio B/A based on TIL and healthy human peripheral blood mononuclear cells was repeated 13 times based on different TIL and healthy human peripheral blood mononuclear cells, as described above. FIG. 8(a) to FIG. 8(c) are diagrams presenting the histograms 71 prepared in 3 procedures among these 13 procedures.

Ratios B/A, an average value, and a standard deviation SD acquired based on 13 biological samples (samples 1 to 13) by 13 procedures are as in the following Table 1. Note that the biological samples presented in FIG. 8(a) to FIG. 8(c) correspond to samples 2, 3 and 6 respectively.

**[Table 1]**

| Biological Sample | Ratio B/A | |
|---|---|---|
| Sample 1 | 1.97 | |
| Sample 2 | 1.94 | |
| Sample 3 | 2.48 | Maximum value |
| Sample 4 | 1.99 | |
| Sample 5 | 2.12 | |
| Sample 6 | 2.03 | |
| Sample 7 | 2.28 | |
| Sample 8 | 2.23 | |
| Sample 9 | 1.67 | |
| Sample 10 | 1.78 | |
| Sample 11 | 2.09 | |
| Sample 12 | 1.35 | Minimum value |
| Sample 13 | 1.57 | |
| Average | 1.96 | |
| SD | 0.31 | |

In relation to the ratios B/A acquired from 13 biological samples, the minimum value was 1.35 and the maximum value was 2.48 as presented in Table 1. In addition, an average of the ratios B/A acquired from 13 biological samples was 1.96, and a standard deviation was an extremely small value of 0.31. In such a way, the inventors found that when the boundary value acquired from lymphocytes (TIL) which infiltrated gastric cancer tissues of patients with gastric cancer was applied to peripheral blood mononuclear cells of healthy humans to acquire the ratios B/A, dispersion of the acquired ratios B/A was reduced, i.e. the substantially equal ratios B/A could be acquired. Also, it was suggested that when the boundary value was set so that the ratios B/A ranged the minimum value 1.35 or more to the maximum value 2.48 or less, cells having FOXP3 levels higher than the boundary value could be judged as the effector type regulatory T cells.

Subsequently, in verification of the inventors, when the ratio B/A was set to the average value 1.96 acquired from Table 1, a reference value was acquired based on the biological samples including peripheral blood mononuclear cells of healthy humans, in the same process as in FIG. 2.

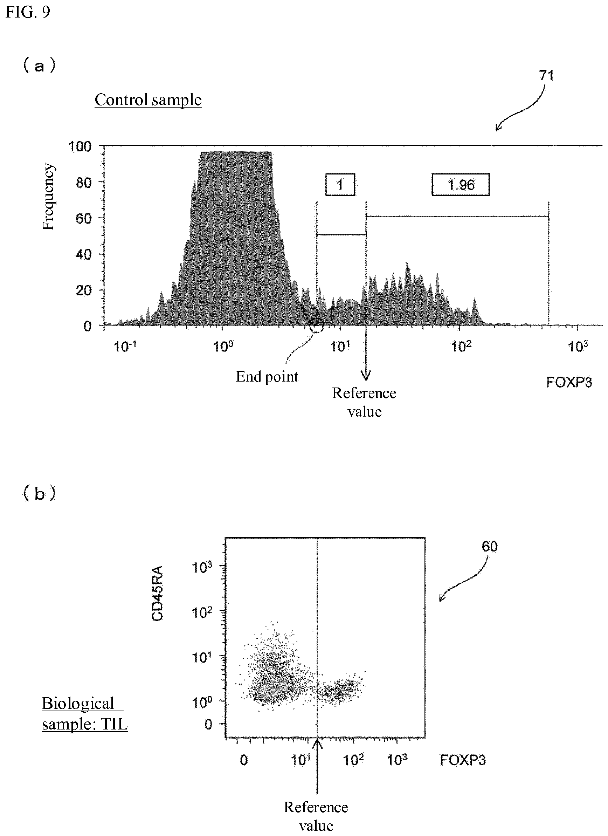

FIG. 9(a) is the histogram 71 acquired by this procedure. In verification of the inventors, the reference value was acquired so that the ratio B/A was 1.96, as presented in FIG. 9(a). In verification of the inventors, the reference value acquired in such a way was applied to the scattergram 60 acquired based on lymphocytes which infiltrated the gastric cancer tissues of patients with gastric cancer, as presented in FIG. 9(b). As presented in FIG. 9(b), according to the reference value acquired in such a way, the FOXP3-negative cell population and the FOXP3-positive cell population can be appropriately fractionated from each other, even when the biological samples are lymphocytes which infiltrate gastric cancer tissues. That means, it was found that when the reference value was acquired based on the ratio B/A set as described above, the effector type regulatory T cells could be appropriately detected from the biological samples collected from the cancer patients.

For the reasons described above, in Embodiment 1, the ratio B/A of 1.35 or more to 2.48 or less, particularly 1.96 is previously stored in the memory section and used in the process for acquiring the reference value based on the control sample.

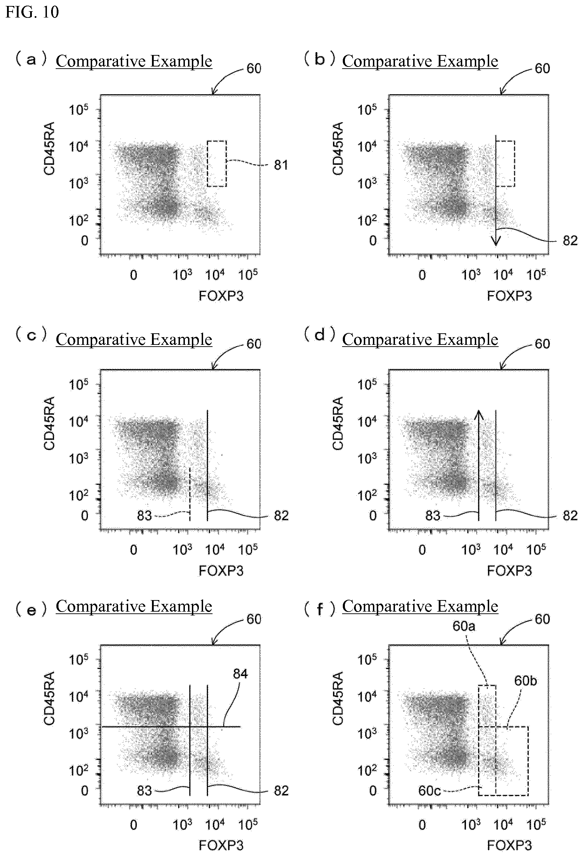

Subsequently, in verification of the inventors, eight biological samples including peripheral blood mononuclear cells of the subjects were compared for the detection accuracy of the effector type regulatory T cells using the ratio B/A of 1.96, based on the procedures in Embodiment 1 and Comparative Example. In Comparative Example, in verification of the inventors, a region of the effector type regulatory T cells was manually fractionated and detected on the scattergram 60. Hereinafter, the manual fractionation carried out by the inventors in Comparative Example will be explained with reference to FIG. 10(a) to FIG. 10(f).

First, a region 81 for excluding a small number of cells on the upper right region is set in a scattergram 60 acquired by representing the CD4-positive T cells, as presented in FIG. 10(a). Then, a boundary line 82 is set downward from the left end of the region 81, as presented in FIG. 10(b). Subsequently, a boundary line 83 is set near the left end of the cell group distributed in the lower right side, as presented in FIG. 10(c). Then, the boundary line 83 is extended upward, as presented in FIG. 10(d). Subsequently, a boundary line 84 extending in a lateral direction is set to fractionate the CD45RA-negative cells and the CD45RA-positive cells, as presented in FIG. 10(e). Finally, regions 60a, 60b and 60c are set based on the boundary lines 82, 83 and 84, as presented in FIG. 10(f). The regions 60a, 60b and 60c are regions corresponding to naive type, effector type and inactive type regulatory T cells respectively. In Comparative Example, cells included in the region 60b are detected as the effector type regulatory T cells.

The following Table 2 presents each proportion of the detected effector type regulatory T cells to the CD4-positive T cells in cases of Embodiment 1 and Comparative Example. Eight biological samples including the peripheral blood mononuclear cells of the subjects are referred to as samples 14 to 21. Table 2 presents an average and a standard deviation SD in Embodiment 1 and an average and a standard deviation SD in Comparative Example.

**[Table 2]**

| Biological Sample | Proportion of eTreg in Embodiment 1 | Proportion of eTreg in Comparative Example |
|---|---|---|
| Sample 14 | 3.22 | 2.93 |
| Sample 15 | 3.64 | 3.35 |
| Sample 16 | 6.96 | 6.98 |
| Sample 17 | 6.14 | 6.14 |
| Sample 18 | 4.18 | 3.73 |
| Sample 19 | 3.39 | 3.06 |
| Sample 20 | 6.08 | 5.89 |
| Sample 21 | 3.25 | 3.05 |
| Average | 4.61 | 4.39 |
| SD | 1.53 | 1.66 |

As presented in Table 2, the proportions of the effector type regulatory T cells were substantially equal between Embodiment 1 and Comparative Example. On the other hand, the standard deviation in Embodiment 1 was lower than in Comparative Example. The above description indicated that, according to Embodiment 1, the effector type regulatory T cells could be detected equivalently to those in Comparative Example, and furthermore dispersion of detection rates of the effector type regulatory T cells was more suppressed than in Comparative Example.

### <Embodiment 2>

In the processing of acquiring a reference value, all the extracted CD4-positive T cells are represented in the histogram 71 in Embodiment 1, but the CD45RA-negative cells among the extracted CD4-positive T cells are represented in the histogram 71 in Embodiment 2.

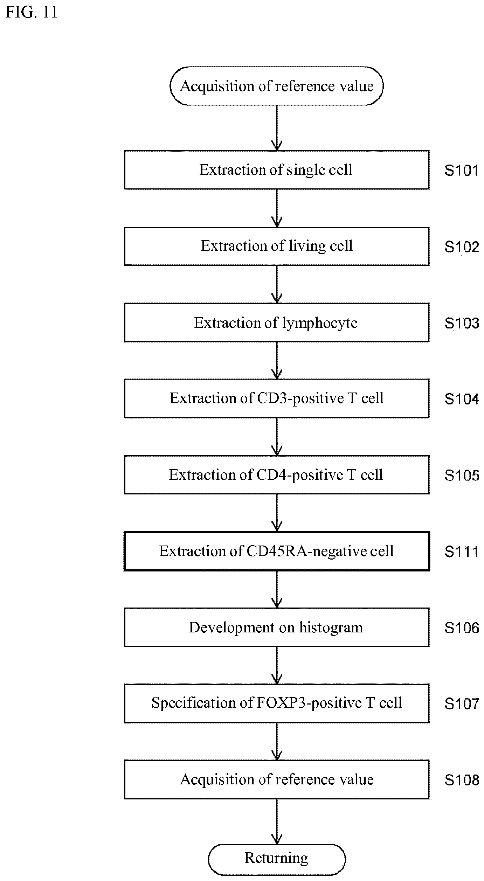

As presented in FIG. 11, in Embodiment 2, step Sill is added between step S106 and step S107 in the process for acquiring the reference value presented in FIG. 2. The other steps in Embodiment 2 are the same as in Embodiment 1. Hereinafter, steps different from the steps in Embodiment 1 will be explained.

In step S111, in the analyzing device, the CD45RA-negative cells are extracted from the CD4-positive T cells extracted in step S105.

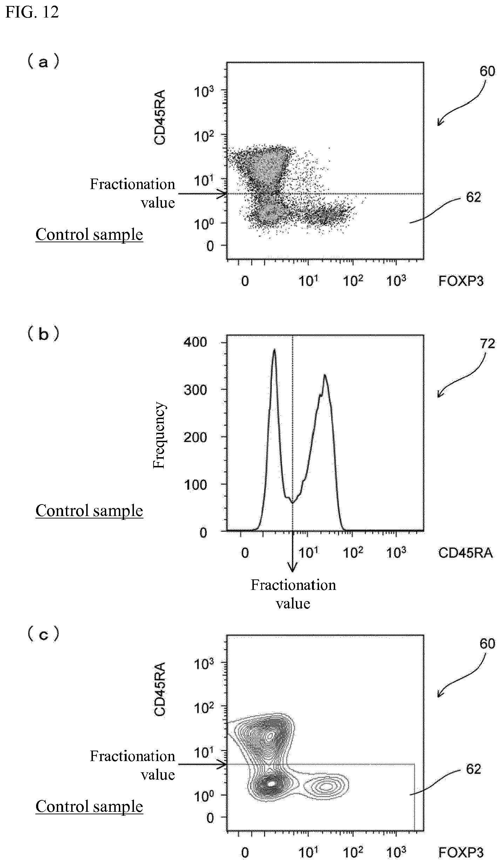

Specifically, on the histogram 71 acquired by representing the CD4-positive T cells, a fractionation value for fractionating the CD45RA-negative cells from the CD45RA-positive cells is set, as presented in FIG. 12(a). In this case, the fractionation value is set in the same manner as in the setting the fractionation value explained with reference to FIG. 6(a) and FIG. 6(b). That means all cells on the scattergram 60 in FIG. 12(a) are represented in the histogram 72 in FIG. 12(b), and the CD45RA level where the frequency is minimum between the peak waveform at the CD45RA-negative side and the peak waveform at the CD45RA-positive side is set as a fractionation value on the histogram 72. The fractionation value set in such a way is applied to the scattergram 60, and a region 62 having a CD45RA level lower than the fractionation value is set on the scattergram 60. In step S111, cells in the region 62 are extracted as the CD45RA-negative cells.

Incidentally, based on contour lines indicating distribution densities of the CD4-positive T cells, a fractionation value may be set between a region having a high distribution density at the CD45RA-positive side and a region having a low distribution density at the CD45RA-negative side, as presented in FIG. 12(c).

Steps S106 to S108 subsequent to step Sill are performed in the same manner as in Embodiment 1. That means, in the analyzing device, the CD45RA-negative cells extracted in step S111 are represented in the histogram 71, in step S106. In the analyzing device, the FOXP3-positive T cells are specified based on the histogram 71 in the same manner as in Embodiment 1, in step S107.

Herein, the FOXP3-positive T cells specified in step S107 of Embodiment 2 are obtained by extracting the CD45RA-negative cells among the FOXP3-positive T cell population extracted in step S107 in FIG. 2. Thus, in Embodiment 2, the order of the FOXP3-positive T cell extraction and the CD45RA-negative cell extraction may be arbitrary. Then, in the analyzing device, a predetermined ratio stored in the memory section is applied to the FOXP3-positive T cell population specified in step S107, and a FOXP3 level which is a division reference for dividing the FOXP3-positive T cell population at this ratio is acquired as a reference value, in step S108.

In such a way, when the reference value is acquired by applying the ratio to the CD45RA-negative population among the FOXP3-positive T cell population, the CD45RA-positive cells as noises are removed, so that the accuracy of the reference value can be further improved.

Incidentally, in Embodiment 2, the process for measuring the biological samples to detect the effector type regulatory T cells is carried out in the same manner as the process in Embodiment 1 presented in FIG. 5. That means, in Embodiment 2, the CD4-positive T cells are extracted in step S205 in FIG. 5, and cells which are CD45RA-negative and have a FOXP3 level higher than the reference value are detected as effector type regulatory T cells in step S206.

In Embodiment 2, the ratio is set so that the ratio B/A of the number B of the cells having the higher FOXP3 level to the number A of the cells having the lower FOXP3 level is 1.62 or more to 2.47 or less. This ratio is a communis ratio found by the inventors, as in Embodiment 1. The reason why the inventors have determined that this ratio is communis will be explained later in verification of Embodiment 2. In Embodiment 2, the ratio B/A is set to 2.01.

Incidentally, the ratio may be set so that a proportion B/(A + B) of the number B of the cells having the higher FOXP3 level to the total cell number (A + B) of the FOXP3-positive T cell population is 62% or more to 72% or less. In this case, a range of the B/A is substantially the same as in the case of the B/A set to 1.62 or more to 2.47 or less, and therefore the reference value can be appropriately set also in this case.

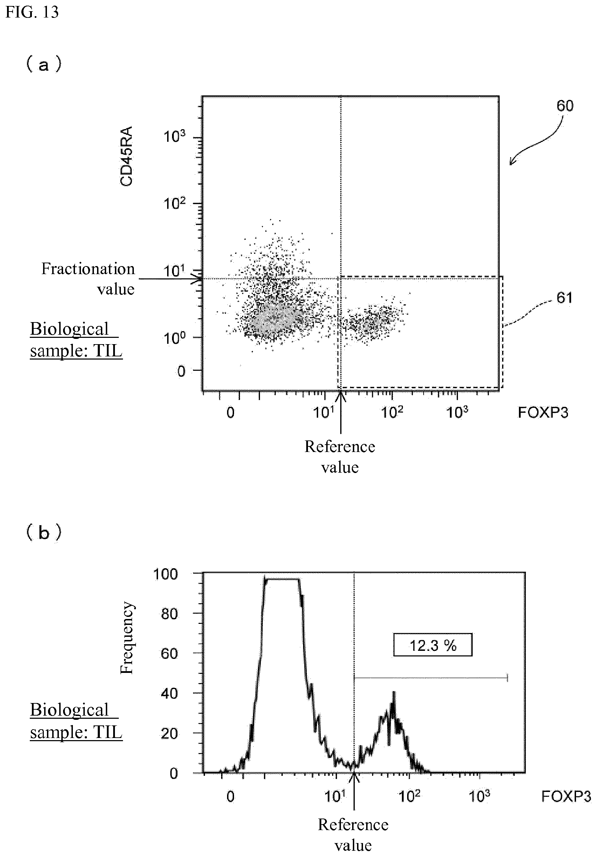

Similarly to Embodiment 1, also in the process for detecting the effector type regulatory T cells in Embodiment 2, the reference value is applied on the scattergram 60, and the fractionation value for fractionating the CD45RA-negative cells and the CD45RA-positive cells is set, as presented in FIG. 13(a). Then, the cells in the region 61 are detected as the effector type regulatory T cells.

In Embodiment 2, as described above, the reference value is acquired based on the CD45RA-negative cells among the CD4-positive T cells, and therefore a more appropriate reference value can be set to detect the effector type regulatory T cells from the biological samples.

The scattergram 60 in FIG. 13(a) is prepared in a case that the biological samples are lymphocytes (TIL) which infiltrate gastric cancer tissues of patients with gastric cancer. Since the biological samples from which the scattergram 60 in FIG. 13(a) is prepared are the same as the biological samples from which the scattergram 60 in FIG. 6(a) is prepared, the cell distributions on the scattergrams 60 in FIG. 13(a) and FIG. 6(a) are the same. On the histogram 71 in FIG. 13(b), all cells distributed on the scattergram 60 in FIG. 13(a) are represented. In the examples presented in FIG. 13(a) and FIG. 13(b), a proportion of the cells having a FOXP3 level higher than the reference value to the entire CD4-positive T cells is 12.3%. This value is substantially equivalent to 12.4% which is the value based on the procedure in Embodiment 1. Thus, also in Embodiment 2, the effector type regulatory T cells can be detected, as in Embodiment 1.

### <Verification of Embodiment 2>

The inventors conducted the same verification as in Embodiment 1 using a result of measuring lymphocytes (TIL) which infiltrate gastric cancer tissues of patients with gastric cancer, and a result of measuring peripheral blood mononuclear cells of healthy humans in the same batch, acquired in verification of Embodiment 1.

In verification of the inventors, a boundary value was visually acquired from the scattergram 60 based on the result of measuring the lymphocytes (TIL) which infiltrates gastric cancer tissues of patients with gastric cancer, as in Embodiment 1. Then, in verification of the inventors, the histogram 71 was prepared by carrying out the same procedures as steps S101 to S105, S111, and S106 in FIG. 11. However, in verification of Embodiment 2, the CD45RA-negative cells are extracted in step S111, and therefore the CD45RA-negative cells among the CD4-positive T cells are represented in the histogram 71. In verification of the inventors, then the acquired boundary value is applied to the histogram 71, and the ratio B/A was calculated based on 13 different biological samples including peripheral blood mononuclear cells of healthy humans.

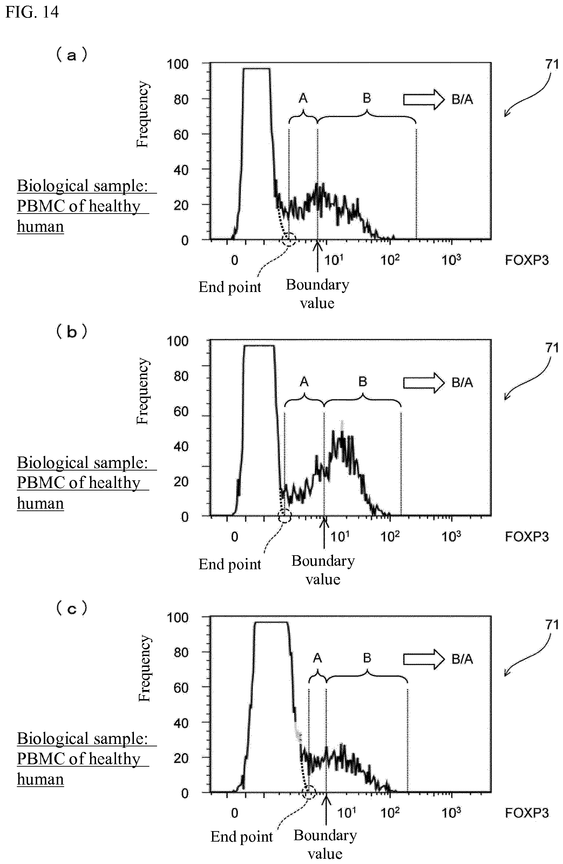

FIG. 14(a) to FIG. 14(c) are diagrams presenting the histograms 71 prepared for three biological samples among these 13 biological samples, and the three biological samples are the same as in FIG. 8(a) to FIG. 8(c) respectively. On the histograms 71 of FIG. 14(a) to FIG. 14(c), not the entire CD4-positive T cells but the CD45RA-negative cells among the CD4-positive T cells are represented, and therefore the shapes in FIG. 14(a) to FIG. 14(c) are different from the shapes in FIG. 8(a) to FIG. 8(c) respectively.

Ratios B/A, an average value, and a standard deviation SD acquired based on 13 biological samples (samples 1 to 13) are as in the following Table 3. Note that the biological samples presented in FIG. 14(a) to FIG. 14(c) correspond to samples 2, 3 and 6 respectively. Samples 1 to 13 are the same as the verification samples 1 to 13 respectively in Embodiment 1.

**[Table 3]**

| Biological Sample | Ratio B/A | |
|---|---|---|
| Sample 1 | 2.21 | |
| Sample 2 | 1.62 | Minimum value |
| Sample 3 | 2.14 | |
| Sample 4 | 1.79 | |
| Sample 5 | 1.95 | |
| Sample 6 | 2.47 | Maximum value |
| Sample 7 | 2.28 | |
| Sample 8 | 2.19 | |
| Sample 9 | 1.63 | |
| Sample 10 | 2.01 | |
| Sample 11 | 2.32 | |
| Sample 12 | 1.68 | |
| Sample 13 | 1.86 | |
| Average | 2.01 | |
| SD | 0.28 | |

In relation to the ratios B/A acquired from 13 biological samples, the minimum value was 1.62 and the maximum value was 2.47 as presented in Table 3. In addition, an average of the ratios B/A acquired from 13 biological samples was 2.01, and a standard deviation was an extremely small value of 0.28. Also in this case, similarly to verification of Embodiment 1, it was found that the dispersion of the acquired ratios B/A was reduced, i.e. the substantially equal ratios B/A could be acquired. Also, it was suggested that when the boundary value was set so that the ratios B/A ranged the minimum value 1.62 or more to the maximum value 2.47 or less, cells having FOXP3 levels higher than the boundary value could be judged as the effector type regulatory T cells.

Subsequently, in verification of the inventors, a reference value was acquired based on the biological samples including peripheral blood mononuclear cells of healthy humans in the same process as in FIG. 11, in a case that the ratio B/A was set to the average value 2.01 acquired from Table 3,

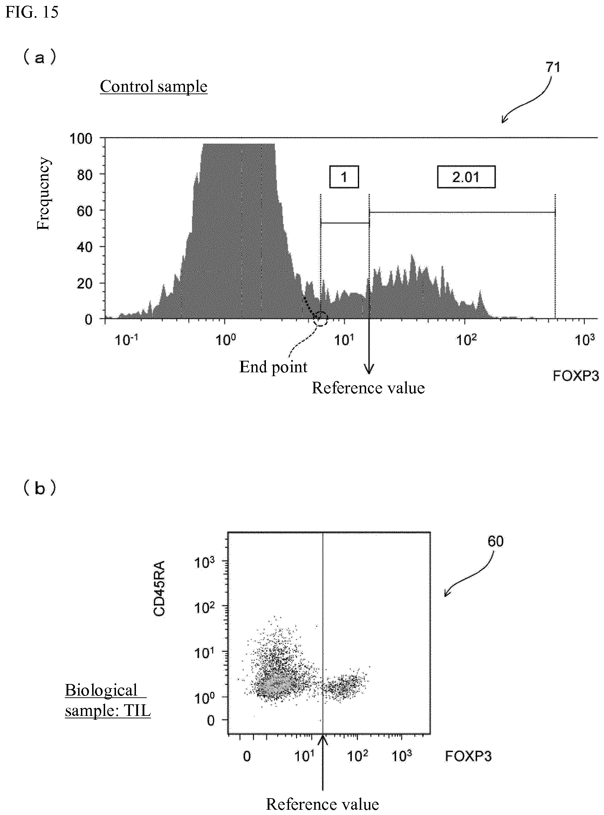

FIG. 15 (a) is the histogram 71 acquired by this procedure. In verification of the inventors, the reference value was acquired so that the ratio B/A was 2.01, as presented in FIG. 15 (a). In verification of the inventors, the reference value acquired in such a way was applied to the scattergram 60 acquired based on lymphocytes which infiltrated the gastric cancer tissues of patients with gastric cancer, as presented in FIG. 15 (b). As presented in FIG. 15 (b), according to the reference value acquired in such a way, the FOXP3-negative cell population and the FOXP3-positive cell population can be fractionated from each other, even when the biological samples are lymphocytes which infiltrate gastric cancer tissues. That means, it was found that when the reference value was acquired based on the ratio B/A set as described above, the effector type regulatory T cells could be appropriately detected from the biological samples collected from the cancer patients.

For the reasons described above, in Embodiment 2, the ratio B/A of 1.62 or more to 2.47 or less, particularly 2.01 is previously stored in the memory section and used in the process for acquiring the reference value based on the control sample.

Subsequently, in verification of the inventors, eight biological samples including peripheral blood mononuclear cells of the subjects were compared for each detection accuracy of the effector type regulatory T cells using the ratio B/A of 2.01, based on the procedures in Embodiment 2 and Comparative Example. In Comparative Example, in verification of the inventors, a region of the effector type regulatory T cells was manually fractionated and detected on the scattergram 60, as explained in verification of Embodiment 1.

The following Table 4 presents each proportion of the detected effector type regulatory T cells to the CD4-positive T cells in cases of Embodiment 2 and Comparative Example. Eight biological samples including peripheral blood mononuclear cells of subjects are referred to as samples 22 to 29. In addition, Table 4 presents an average and a standard deviation SD in Embodiment 2 and an average and a standard deviation SD in Comparative Example.

**[Table 4]**

| Biological Sample | Proportion of eTreg in Embodiment 2 | Proportion of eTreg in Comparative Example |
|---|---|---|
| Sample 22 | 3.17 | 3.12 |
| Sample 23 | 3.54 | 3.34 |
| Sample 24 | 5.89 | 5.89 |
| Sample 25 | 6.69 | 6.36 |
| Sample 26 | 4.21 | 4.06 |
| Sample 27 | 3.57 | 3.20 |
| Sample 28 | 5.50 | 5.50 |
| Sample 29 | 2.69 | 2.33 |
| Average | 4.41 | 4.23 |
| SD | 1.44 | 1.49 |

As presented in Table 4, the proportions of the effector type regulatory T cells were substantially equal between Embodiment 2 and Comparative Example. On the other hand, the standard deviation in Embodiment 2 was lower than in Comparative Example. The above description indicated that, according to Embodiment 2, the effector type regulatory T cells could be detected equivalently to those in Comparative Example, and furthermore dispersion of detection rates of the effector type regulatory T cells was more suppressed than in Comparative Example.

### <Embodiment 3>

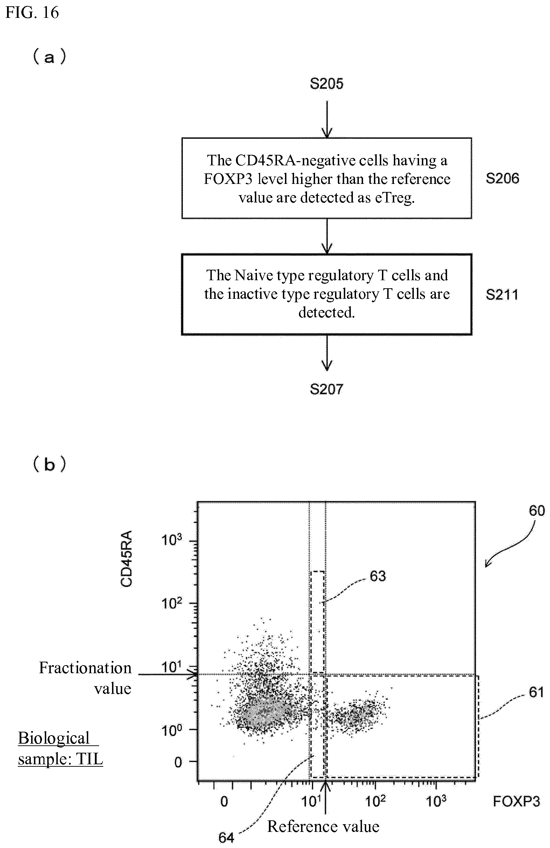

As presented in FIG. 16(a), in the process for detecting the effector type regulatory T cells in Embodiment 3, step S211 is added between step S206 and step S207, as compared to FIG. 5. Other configurations in Embodiment 3 are the same as those in Embodiment 1 or 2.

In step S211, in the analyzing device, naive type regulatory T cells and inactive type regulatory T cells are detected.

Specifically, in the analyzing device, regions 63 and 64 are set on the scattergram 60 acquired by representation in step S206, as presented in FIG. 16(b). The region 63 is a region where the FOXP3 level is lower than the reference value and higher than the FOXP3 level at the end point, and the CD45RA level is higher than the fractionation value. The region 64 is a region where the FOXP3 level is lower than the reference value and higher than the FOXP3 level at the end point, and the CD45RA level is lower than the fractionation value. Herein, the reference value is the same as the reference value used in step S206. When the scattergram 60 is represented in the histogram 71, the end point is a point prepared in the same manner as in the procedure explained in FIG. 4, and is a point in the higher value side of the peak waveform in the FOXP3-negative side of the histogram 71. The fractionation value refers to a CD45RA level for distinguishing CD45RA-negative cells and CD45RA-positive cells, as explained in FIG. 6(a).

In step S211, in the analyzing device, the cells included in the regions 63 and 64 on the scattergram 60 are detected as the naive type regulatory T cells and the inactive type regulatory T cells respectively. Subsequently in step S207, in the analyzing device, information about the effector type regulatory T cells, the naive type regulatory T cells, and the inactive type regulatory T cells detected in steps S206 and S207 is displayed on the display section. In step S207, the number of each of the aforementioned cells, and the proportion of each of the aforementioned cells to the entire CD4-positive T cells are displayed.

### <Analyzing System>

FIG. 17 is a block diagram presenting a configuration of an analyzing system 100 for carrying out the analyses explained in Embodiments 1 to 3.

The analyzing system 100 includes a labeling device 110 and an analyzing device 120 which is a flow cytometer. The labeling device 110 fluorescently labels the test substances in a control sample 101 and a biological sample 102. The labeling device 110 allows automization of the fluorescent labeling for the test substances in the control sample 101 and the biological sample 102. Thereby, a plurality of samples can be prepared under uniform conditions. In the analyzing device 120, the control sample 101 and the biological sample 102 are analyzed. The analyzing device 120 includes a measurement unit 200 as a measurement section for measurement, and an analyzing unit 300 as an analyzing section for analysis.

Incidentally, the labeling device 110 and the analyzing device 120 are not necessarily separated, e.g. the analyzing device 120 and the labeling device 110 may be integrally configured. In addition, when the analyzing system 100 includes a device for purifying peripheral blood mononuclear cells from whole peripheral blood on a preceding stage of the labeling device 110, the control sample 101 and the biological sample 102 may be whole peripheral blood.

When the processings presented in FIG. 1(a) and FIG. 1(c) are executed by the analyzing system 100, step S11 in FIG. 1(a) and step S21 in FIG. 1(c) are executed by the labeling device 110. Step S12 in FIG. 1(a) and step S22 in FIG. 1(c) are executed by the measurement unit 200 of the analyzing device 120. Step S13 in FIG. 1(a) and step S23 in FIG. 1(c) are executed by the analyzing unit 300 of the analyzing device 120.

The measurement unit 200 includes a measurement controlling section 210, a detecting section 220, and a signal processing circuit 230. The measurement controlling section 210 is composed of e.g. a CPU, an MPU, and the like. The measurement controlling section 210 receives signals output from each section in the measurement unit 200 and controls each section in the measurement unit 200. In the detecting section 220, the control sample 101 and the biological sample 102 which have been labeled in the labeling device 110 is caused to flow into a flow cell, the flow cell is irradiated with light, and lights generated from the samples are received by a light receiving section to output signals corresponding to light intensities.

In the signal processing circuit 230, signals output from the detecting section 220 are processed. Specifically in the signal processing circuit 230, waveform signals for each particle are extracted based on the signals output from the light receiving section of the detecting section 220 to calculate feature parameters such as a peak value, an area and a width from the waveform signals for each particle. The measurement controlling section 210 communicates with the analyzing unit 300. By the measurement controlling section 210, the feature parameters as measurement results for each particle calculated by the signal processing circuit 230 are transmitted to the analyzing unit 300.

The analyzing unit 300 includes a control section 310, a memory section 320, a display section 330, and an input section 340. The control section 310 is composed of e.g. a CPU. The control section 310 receives signals output from each section in the analyzing unit 300 and controls each section in the analyzing unit 300. The control section 310 communicates with the measurement unit 200. The memory section 320 is composed of e.g. a ROM, a RAM, a hard disk, and the like. The control section 310 executes the processing based on the program 321 stored in the memory section 320. The program 321 includes a program for executing the processing presented in FIG. 1(a) and FIG. 1(c).

The display section 330 is composed of e.g. a display. The display section 330 displays a screen indicating analysis results. In the displaying processing of step S207 in FIG. 5, in the control section 310, information about the detected regulatory T cells is displayed on the display section 330. The input section 340 is composed of e.g. a mouse, a keyboard, and the like. The input section 340 receives input from the operator. The display section 330 and the input section 340 may be integrally configured by means of a touch panel or the like.

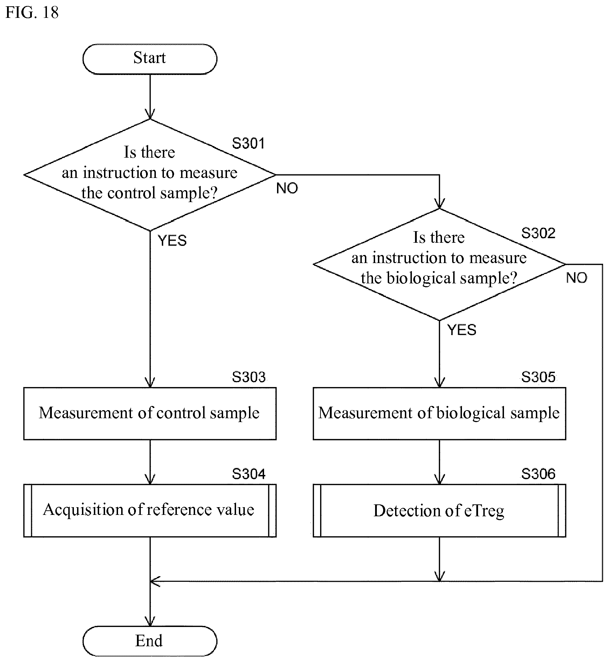

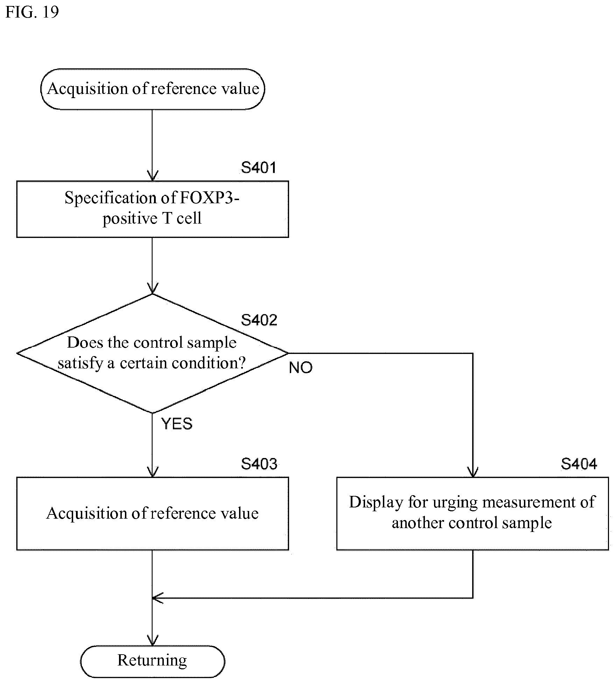

FIG. 18 and FIG. 19 are flowcharts presenting processings which are execute by the analyzing device 120.

As presented in FIG. 18, in steps S301 and S302, the control section 310 judges whether or not an instruction to measure the control sample 101 or the biological sample 102 which has been labeled by the labeling device 110 is input by an operator via the input section 340. When the instruction to measure the control sample 101 is input, the processing proceeds to step S303, and when the instruction to measure the biological sample 102 is input, the processing proceeds to step S305. When no measurement instruction is input, the processing is completed, and the processing starts again from step S301.

When the instruction to measure the control sample 101 is input, the measurement controlling section 210 measures the control sample 101 in step S303, in the same manner as in step S12 in FIG. 1(a). The measurement controlling section 210 transmits a result of measuring the control sample 101 to the control section 310. In the control section 310, the control sample 101-measuring result received from the measurement controlling section 210 is stored in the memory section 320. Subsequently, in step S304, in the control section 310, the result of measuring the control sample 101 is read out from the memory section 320 to acquire a reference value based on the read measurement result. At this time, in the control section 310, the acquired reference value is stored in the memory section 320. The processing for acquiring the reference value in this case will be explained with reference to FIG. 19 later.

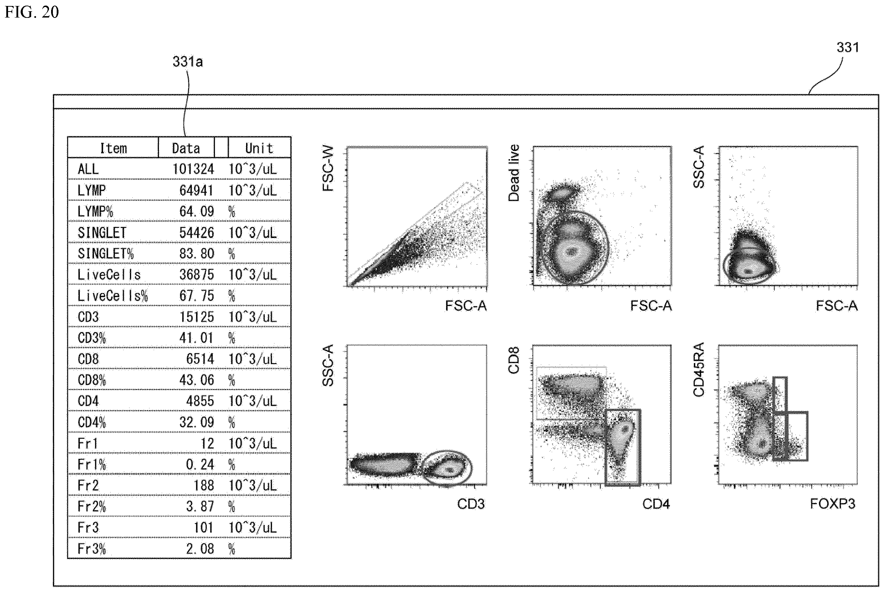

When the instruction to measure the biological sample 102 is input, the measurement controlling section 210 measures the biological sample 102 in step S305, in the same manner as in step S22 in FIG. 1(c). Also in this case, the measurement controlling section 210 transmits the result of measuring the biological sample 102 to the control section 310. In the control section 310, the biological sample 102-measuring result received from the measurement controlling section 210 is stored in the memory section 320. Subsequently, in step S306, in the control section 310, the result of measuring the biological sample 102 is read out from the memory section 320, and based on the read measurement result, the effector type regulatory T cells are detected in the same manner as in the processing presented in FIG. 5 or FIG. 16(a). At this time, in the control section 310, the reference value stored in step S304 is read out from the memory section 320, and the read reference value is applied to detection of the effector type regulatory T cells in the analysis of the biological sample 102. In the display processing executed in step S306, the screen presented in FIG. 20 is displayed. The screen presented in FIG. 20 will be explained later.

As presented in FIG. 19, in step S401, the control section 310 specifies the FOXP3-positive T cells contained in the control sample 101. In step S401, the same processings as in steps S101 to S107 in FIG. 2 are executed. Thereby, on the histogram 71 acquired by representing the CD4-positive T cells, a cell population having a FOXP3 level equal to or higher than the FOXP3 level at the end point is specified as the FOXP3-positive T cells. In step S401, the same processing as in steps S101 to S105, S111, S106, and S107 in FIG. 11 may be executed. In this case, the CD4-positive and CD45RA-negative cell population is specified as the FOXP3-positive T cells.

Subsequently, in step S402, the control section 310 judges whether or not the control sample 101 satisfies a predetermined condition. The predetermined condition is previously stored in the memory section 320. The predetermined condition is e.g. a condition that the number of the FOXP3-positive T cells is within a predetermined range. In this case, when the number of the FOXP3-positive T cells specified in step S401 is within a predetermined range, the control section 310 judges that the control sample 101 satisfies a predetermined condition.

The predetermined condition in step S402 is not limited to the above description, and may also be a requirement that the number of the FOXP3-negative T cells is within a predetermined range, a requirement that a ratio of the CD45RA-positive T cells to the CD45RA-negative T cells is within a predetermined range, and a requirement that the number of the naive type regulatory T cells is within a predetermined range. The predetermined condition may also be a requirement satisfying a plurality of conditions among these conditions. The FOXP3-negative T cell population is specified as e.g. a cell population having a FOXP3 level lower than a FOXP3 level at the end point on the histogram 71. The CD45RA-positive T cell population is specified as e.g. the cell population in the region 62 in FIG. 12(a), and the CD45RA-negative T cell population is specified as e.g. a cell population having a CD45RA level higher than the fractionation value in FIG. 12(a). The naive type regulatory T cell population is specified as e.g. the cell population in the region 63 presented in FIG. 16(b).

When the control sample 101 satisfies a predetermined condition, the control section 310 acquires a reference value based on the histogram 71 acquired from the control sample 101 in step S403, in the same manner as in step S108 in FIG. 2 or FIG. 11. Then, in the control section 310, the acquired reference value is stored in the memory section 320. On the other hand, when the control sample 101 does not satisfy the predetermined condition, a screen for urging the operator to measure another control sample 101 instead of the control sample 101 supporting judgement in step S402 is displayed on the display section 330 in the control section 310 in step S404. Thereby, the operator can recognize that the control sample 101 is not suitable as a sample for acquiring the reference value. Then, the operator inputs an instruction to measure the control sample again, using another control sample 101.

FIG. 20 presents a screen 331 displayed on the display section 330 in the processing of detecting the effector type regulatory T cells presented in step S306 in FIG. 18. Incidentally, the screen 331 in FIG. 20 displays information about the naive type regulatory T cells and the inactive type regulatory T cells in addition to information about the effector type regulatory T cells. That means, the screen 331 illustrated in FIG. 20 is a screen in a case of executing the detection processing of embodiment 3 presented in FIG. 16(a) as step S306 in FIG. 18.

The screen 331 is a screen indicating the result of analyzing the biological sample 102, and includes a list region 331a indicating the analysis value, and scattergrams having the same axes as in FIG. 3(a) to FIG. 3(f). The list region 331a displays numbers and ratios of extracted cells. Fr1 indicates a number of cells in the region 63 in FIG. 16(b) i.e. a number of the naive type regulatory T cells. Fr2 indicates a number of cells in the region 61 in FIG. 16(b) i.e. a number of the effector type regulatory T cells. Fr3 indicates a number of cells in the region 64 in FIG. 16(b) i.e. a number of the inactive type regulatory T cells. Fr1%, Fr2%, and Fr3% represent ratios of the naive type regulatory T cells, the effector type regulatory T cells, and the inactive type regulatory T cells respectively to the CD4-positive T cells. The screen 331 is displayed on the display section 330, so that the operator can visually comprehend the number and ratio of each cell, and the generated scattergram.

Incidentally, in the control section 310, it is also possible to judge whether or not the biological sample 102 has a pharmacological effect as a cancer drug in analysis of the biological sample 102, and to display information indicating whether or not there is the pharmacological effect as the cancer drug on the screen 331.

Furthermore, in the control section 310, information about the control samples 101 measured in the same batch may be displayed on the display section 330 in accordance with a display instruction from the operator. As information about the control sample 101, e.g. the histogram 71 acquired from the control sample 101, and a reference value acquired from the histogram 71 are displayed. As a result, the operator can determine whether the reference value has been appropriate.

### Reference Signs List

- 71: Histogram
- 100: Analyzing system
- 101: Control sample
- 102: Biological Sample
- 110: Labeling device
- 120: Analyzing device, Flow cytometer
- 200: Measurement unit, Measurement section
- 220: Flow cytometer
- 310: Control section
- 320: Memory section
- 321: Program

## Claims

1. An effector type regulatory T cell detecting method using flow cytometry, the effector type regulatory T cell detecting method comprising:
measuring a control sample containing a CD4-positive T cell population;
acquiring, based on a result of measuring the control sample, a FOXP3 level being a division reference for dividing a FOXP3-positive T cell population contained in the CD4-positive T cell population at a predetermined ratio, as a reference value to be used for measuring a biological sample;
measuring a biological sample containing a CD4-positive T cell population; and
detecting, based on a result of measuring the biological sample, effector type regulatory T cells from a cell population having a FOXP3 level higher than the reference value.

2. The effector type regulatory T cell detecting method according to claim 1, wherein the control sample is measured for each measurement of one biological sample to acquire the reference value, and the acquired reference value is used for detecting the effector type regulatory T cells in the measurement of the one biological sample.

3. The effector type regulatory T cell detecting method according to claim 1, wherein the control sample is measured for each measurement of a plurality of biological samples to acquire the reference value, and the acquired reference value is used for detecting the effector type regulatory T cells in the measurement of each biological sample.

4. The effector type regulatory T cell detecting method according to claim 2 or 3, wherein the reference value is acquired by applying the ratio to an entire FOXP3-positive T cell population acquired based on the result of measuring the control sample.

5. The effector type regulatory T cell detecting method according to claim 4, wherein the ratio is set so that a proportion of a number of cells having a higher FOXP3 level to a total cell number of the FOXP3-positive T cell population acquired based on the result of measuring the control sample is 57% or more to 72% or less.

6. The effector type regulatory T cell detecting method according to claim 4, wherein the ratio is set so that a ratio of a number of cells having the higher FOXP3 level relative to a number of cells having a lower FOXP3 level is 1.35 or more to 2.48 or less in the FOXP3-positive T cell population acquired based on the result of measuring the control sample.

7. The effector type regulatory T cell detecting method according to any one of claims 4 to 6, wherein when the CD4-positive T cell population acquired based on the result of measuring the control sample is represented in a histogram with an axis of FOXP3 level, a cell population having a FOXP3 level equal to or higher than a FOXP3 level at an end point of a peak waveform in a FOXP3-negative side of the histogram is specified as the FOXP3-positive T cell population.

8. The effector type regulatory T cell detecting method according to claim 7, wherein when an approximated curve is applied to the peak waveform in the FOXP3-negative side of the histogram, a point at which the approximated curve intersects with the axis in a higher value side of the FOXP3 level is set as the end point.

9. The effector type regulatory T cell detecting method according to claim 2 or 3, wherein the reference value is acquired by applying the ratio to a CD45RA-negative population among the FOXP3-positive T cell population acquired based on the result of measuring the control sample.

10. The effector type regulatory T cell detecting method according to claim 9, wherein the ratio is set so that a proportion of a number of cells having a higher FOXP3 level to a total cell number of the FOXP3-positive T cell population acquired based on the result of measuring the control sample is 62% or more to 72% or less.

11. The effector type regulatory T cell detecting method according to claim 9, wherein the ratio is set so that the ratio of a number of cells having a higher FOXP3 level relative to a number of cells having a lower FOXP3 level is 1.62 or more to 2.47 or less in the FOXP3-positive T cell population acquired based on the result of measuring the control sample.

12. The effector type regulatory T cell detecting method according to any one of claims 9 to 11, wherein when the CD45RA-negative cell population acquired based on the result of measuring the control sample is represented in a histogram with an axis of the FOXP3 level, a cell population having a FOXP3 level equal to or higher than a FOXP3 level at an end point of a peak waveform in a FOXP3-negative side of the histogram is specified as the FOXP3-positive T cell population.

13. The effector type regulatory T cell detecting method according to claim 12, wherein when an approximated curve is applied to the peak waveform in the FOXP3-negative side of the histogram, a point at which the approximated curve intersects with the axis in a higher value side of the FOXP3 level is set as the end point.

14. The effector type regulatory T cell detecting method according to any one of claims 1 to 13, wherein based on the result of measuring the biological sample, a cell population which is CD45RA-negative and has a FOXP3 level higher than the reference value is detected as the effector type regulatory T cells.

15. The effector type regulatory T cell detecting method according to claim 14, wherein, based on the result of measuring the biological sample,
a single living lymphocyte population is extracted;
a CD3-positive cell population is extracted from the extracted lymphocyte population;
a CD4-positive cell population is extracted from the extracted CD3-positive cell population; and
a cell population which is CD45RA-negative and has a FOXP3 level higher than the reference value among the extracted CD4-positive cell population, is detected as the effector type regulatory T cells.

16. The effector type regulatory T cell detecting method according to any one of claims 1 to 15, wherein the control sample is a sample containing a peripheral blood of a healthy human, or peripheral blood mononuclear cells purified from a peripheral blood of a healthy human.

17. An effector type regulatory T cell analyzing device using flow cytometry, the effector type regulatory T cell analyzing device comprising:
a measurement section for measuring a biological sample containing a CD4-positive T cell population; and
a control section which is configured,
based on a result of measuring a control sample containing a CD4-positive T cell population by the measurement section, to acquire a FOXP3 level being a division reference for dividing a FOXP3-positive T cell population contained in the CD4-positive T cell population at a predetermined ratio as a reference value to be used for measuring the biological sample, and
based on a result of measuring the biological sample containing the CD4-positive T cell population by the measurement section, to detect effector type regulatory T cells from a cell population having a FOXP3 level higher than the reference value.

18. An effector type regulatory T cell analyzing system comprising:
the analyzing device according to claim 17; and
a labeling device for fluorescently labeling test substances in the control sample and the biological sample.

## Patentansprüche

1. Verfahren zur Detektion einer regulatorischen T-Zelle vom Effektortyp unter Verwendung von Durchflusszytometrie, wobei das Verfahren zur Detektion einer regulatorischen T-Zelle vom Effektortyp umfasst:
Messen einer Kontrollprobe, die eine CD-4-positive T-Zell-Population enthält;
Erfassen, auf Grundlage eines Ergebnisses des Messens der Kontrollprobe, eines FOXP3-Spiegels, der eine Teilungsreferenz zum Teilen einer FOXP3-positiven T-Zell-Population ist, die in der CD-4-positiven T-Zell-Population in einem vorher festgelegten Verhältnis enthalten ist, als einen zum Messen einer biologischen Probe zu verwendenden Referenzwert;
Messen einer biologischen Probe, die eine CD-4-positive T-Zell-Population enthält; und
Detektieren, auf Grundlage eines Ergebnisses des Messens der biologischen Probe, von regulatorischen T-Zellen vom Effektortyp aus einer Zellpopulation, die einen FOXP3-Spiegel höher als der Referenzwert aufweist.

2. Verfahren zur Detektion einer regulatorischen T-Zelle vom Effektortyp nach Anspruch 1, wobei die Kontrollprobe für jede Messung von einer biologischen Probe gemessen wird, um den Referenzwert zu erfassen, und der erfasste Referenzwert zum Detektieren der regulatorischen T-Zellen vom Effektortyp bei der Messung der einen biologischen Probe verwendet wird.

3. Verfahren zur Detektion einer regulatorischen T-Zelle vom Effektortyp nach Anspruch 1, wobei die Kontrollprobe für jede Messung einer Vielzahl von biologischen Proben gemessen wird, um den Referenzwert zu erfassen, und der erfasste Referenzwert zum Detektieren der regulatorischen T-Zellen vom Effektortyp bei der Messung von jeder biologischen Probe verwendet wird.

4. Verfahren zur Detektion einer regulatorischen T-Zelle vom Effektortyp nach Anspruch 2 oder 3, wobei der Referenzwert durch Anwenden des Verhältnisses auf eine gesamte FOXP3-positive T-Zell-Population erfasst wird, die auf Grundlage des Ergebnisses des Messens der Kontrollprobe erhalten wird.

5. Verfahren zur Detektion einer regulatorischen T-Zelle vom Effektortyp nach Anspruch 4, wobei das Verhältnis so eingestellt wird, dass ein Mengenverhältnis einer Anzahl von Zellen, die einen höheren FOXP3-Spiegel aufweisen, zu einer Gesamtzellanzahl der auf Grundlage des Ergebnisses des Messens der Kontrollprobe erfassten FOXP3-positiven T-Zell-Population 57 % oder mehr bis 72 % oder weniger beträgt.

6. Verfahren zur Detektion einer regulatorischen T-Zelle vom Effektortyp nach Anspruch 4, wobei das Verhältnis so eingestellt wird, dass ein Verhältnis einer Anzahl von Zellen, die den höheren FOXP3-Spiegel aufweisen, relativ zu einer Anzahl von Zellen, die einen niedrigeren FOXP3-Spiegel aufweisen, in der auf Grundlage des Ergebnisses des Messens der Kontrollprobe erfassten FOXP3-positiven T-Zell-Population 1,35 oder mehr bis 2,48 oder weniger beträgt.

7. Verfahren zur Detektion einer regulatorischen T-Zelle vom Effektortyp nach einem der Ansprüche 4 bis 6, wobei, wenn die auf Grundlage des Ergebnisses des Messens der Kontrollprobe erfasste CD4-positive T-Zell-Population in einem Histogramm mit einer FOXP3-Spiegel-Achse dargestellt wird, eine Zellpopulation mit einem FOXP3-Spiegel gleich oder höher als ein FOXP3-Spiegel an einem Endpunkt einer Peakwellenform auf einer FOXP3-negativen Seite des Histogramms als die FOXP3-positive T-Zell-Population spezifiziert wird.

8. Verfahren zur Detektion einer regulatorischen T-Zelle vom Effektortyp nach Anspruch 7, wobei, wenn eine angenäherte Kurve auf die Peakwellenform auf der FOXP3-negativen Seite des Histogramms angewendet wird, ein Punkt, an dem sich die angenäherte Kurve mit der Achse auf einer Seite mit höherem Wert des FOXP3-Spiegels schneidet, als der Endpunkt eingestellt wird.

9. Verfahren zur Detektion einer regulatorischen T-Zelle vom Effektortyp nach Anspruch 2 oder 3, wobei der Referenzwert durch Anwenden des Verhältnisses auf eine CD45RA-negative Population inmitten der FOXP3-positiven T-Zell-Population erfasst wird, die auf Grundlage des Ergebnisses des Messens der Kontrollprobe erfasst wird.

10. Verfahren zur Detektion einer regulatorischen T-Zelle vom Effektortyp nach Anspruch 9, wobei das Verhältnis so eingestellt wird, dass ein Mengenverhältnis einer Anzahl von Zellen, die einen höheren FOXP3-Spiegel aufweisen, zu einer Gesamtzellanzahl der auf Grundlage des Ergebnisses des Messens der Kontrollprobe erfassten FOXP3-positiven T-Zell-Population 62 % oder mehr bis 72 % oder weniger beträgt.

11. Verfahren zur Detektion einer regulatorischen T-Zelle vom Effektortyp nach Anspruch 9, wobei das Verhältnis so eingestellt wird, dass das Verhältnis einer Anzahl von Zellen, die einen höheren FOXP3-Spiegel aufweisen, relativ zu einer Anzahl von Zellen, die einen niedrigeren FOXP3-Spiegel aufweisen, in der auf Grundlage des Ergebnisses des Messens der Kontrollprobe erfassten FOXP3-positiven T-Zell-Population 1,62 oder mehr bis 2,47 oder weniger beträgt.

12. Verfahren zur Detektion einer regulatorischen T-Zelle vom Effektortyp nach einem der Ansprüche 9 bis 11, wobei, wenn die auf Grundlage des Ergebnisses des Messens der Kontrollprobe erfasste CD45RA-negative Zellpopulation in einem Histogramm dargestellt wird mit einer Achse des FOXP3-Spiegels, eine Zellpopulation, die einen FOXP3-Spiegel gleich oder höher als ein FOXP3-Spiegel aufweist, an einem Endpunkt einer Peakwellenform auf einer FOXP3-negativen Seite des Histogramms als die FOXP3-positive T-Zell-Population spezifiziert wird.

13. Verfahren zur Detektion einer regulatorischen T-Zelle vom Effektortyp nach Anspruch 12, wobei, wenn eine angenäherte Kurve auf die Peakwellenform auf der FOXP3-negativen Seite des Histogramms angewendet wird, ein Punkt, an dem sich die angenäherte Kurve mit der Achse auf einer Seite mit höherem Wert des FOXP3-Spiegels schneidet, als der Endpunkt eingestellt wird.

14. Verfahren zur Detektion einer regulatorischen T-Zelle vom Effektortyp nach einem der Ansprüche 1 bis 13, wobei auf Grundlage des Ergebnisses des Messens der biologischen Probe eine Zellpopulation, die CD45RA-negativ ist und einen FOXP3-Spiegel aufweist, der höher als der Referenzwert ist, als die regulatorischen T-Zellen vom Effektortyp detektiert wird.

15. Verfahren zur Detektion einer regulatorischen T-Zelle vom Effektortyp nach Anspruch 14, wobei, auf Grundlage des Ergebnisses des Messens der biologischen Probe,
eine einzelne lebende Lymphozytenpopulation extrahiert wird;
eine CD3-positive Zellpopulation aus der extrahierten Lymphozytenpopulation extrahiert wird;
eine CD4-positive Zellpopulation aus der extrahierten CD3-positiven Zellpopulation extrahiert wird; und
eine Zellpopulation, die CD45RA-negativ ist und einen FOXP3-Spiegel höher als der Referenzwert inmitten der extrahierten CD4-positiven Zellpopulation aufweist, als die regulatorischen T-Zellen vom Effektortyp detektiert wird.

16. Verfahren zur Detektion einer regulatorischen T-Zelle vom Effektortyp nach einem der Ansprüche 1 bis 15, wobei die Kontrollprobe eine Probe ist, die peripheres Blut eines gesunden Menschen oder periphere mononukleare Blutzellen, gereinigt aus einem peripheren Blut eines gesunden Menschen, enthält.

17. Analysevorrichtung für regulatorische T-Zelle vom Effektortyp unter Verwendung von Durchflusszytometrie, wobei die Analysiervorrichtung für regulatorische T-Zelle vom Effektortyp umfasst:
einen Messabschnitt zum Messen einer biologischen Probe, die eine CD4-positive T-Zell-Population enthält;
einen Kontrollabschnitt, der konfiguriert ist, um auf Grundlage eines Ergebnisses des Messens einer Kontrollprobe durch den Messabschnitt, die eine CD4-positive T-Zell-Population enthält, einen FOXP3-Spiegel, der eine Teilungsreferenz zum Teilen einer FOXP3-positiven T-Zell-Population ist, die in der CD4-positiven T-Zell-Population bei einem vorher festgelegten Verhältnis enthalten ist, als einen zum Messen einer biologischen Probe zu verwendenden Referenzwert zu erfassen, und
auf Grundlage eines Ergebnisses des Messens der biologischen Probe durch den Messabschnitt, die die CD4-positive T-Zell-Population enthält, regulatorische T-Zellen vom Effektortyp aus einer Zellpopulation mit einem FOXP3-Spiegel höher als der Referenzwert zu detektieren.

18. Analysesystem für regulatorische T-Zelle vom Effektortyp, umfassend:
die Analysevorrichtung nach Anspruch 17; und
eine Markierungsvorrichtung zum Fluoreszenzmarkieren von Prüfsubstanzen in der Kontrollprobe und der biologischen Probe.

## Revendications

1. Procédé de détection de lymphocytes T régulateurs de type effecteur utilisant la cytométrie en flux, le procédé de détection de lymphocytes T régulateurs de type effecteur comprenant :
la mesure d'un échantillon témoin contenant une population de lymphocytes T CD4-positifs ;
l'acquisition, sur la base d'un résultat de mesure de l'échantillon témoin, d'un niveau de FOXP3 qui est une référence de division pour diviser une population de lymphocytes T FOXP3-positifs contenue dans la population de lymphocytes T CD4-positifs à un rapport prédéterminé, en tant que valeur de référence à utiliser pour mesurer un échantillon biologique ;
la mesure d'un échantillon biologique contenant une population de lymphocytes T CD4-positifs ; et
la détection, sur la base d'un résultat de mesure de l'échantillon biologique, de lymphocytes T régulateurs de type effecteur parmi une population de cellules présentant un niveau de FOXP3 supérieur à la valeur de référence.

2. Procédé de détection de lymphocytes T régulateurs de type effecteur selon la revendication 1, dans lequel l'échantillon témoin est mesuré pour chaque mesure d'un échantillon biologique pour acquérir la valeur de référence, et la valeur de référence acquise est utilisée pour détecter les lymphocytes T régulateurs de type effecteur dans la mesure de l'échantillon biologique.

3. Procédé de détection de lymphocytes T régulateurs de type effecteur selon la revendication 1, dans lequel l'échantillon témoin est mesuré pour chaque mesure d'une pluralité d'échantillons biologiques pour acquérir la valeur de référence, et la valeur de référence acquise est utilisée pour détecter les lymphocytes T régulateurs de type effecteur dans la mesure de chaque échantillon biologique.

4. Procédé de détection de lymphocytes T régulateurs de type effecteur selon la revendication 2 ou 3, dans lequel la valeur de référence est acquise en appliquant le rapport à une population entière de lymphocytes T FOXP3-positifs acquise sur la base du résultat de la mesure de l'échantillon témoin.

5. Procédé de détection de lymphocytes T régulateurs de type effecteur selon la revendication 4, dans lequel le rapport est établi de sorte qu'une proportion d'un nombre de cellules présentant un niveau de FOXP3 plus élevé par rapport à un nombre total de cellules de la population de lymphocytes T FOXP3-positifs acquise sur la base du résultat de mesure de l'échantillon témoin est de 57 % ou plus à 72 % ou moins.

6. Procédé de détection de lymphocytes T régulateurs de type effecteur selon la revendication 4, dans lequel le rapport est établi de sorte qu'un rapport d'un nombre de cellules présentant le niveau de FOXP3 plus élevé par rapport à un nombre de cellules présentant un niveau de FOXP3 plus bas est de 1,35 ou plus à 2,48 ou moins dans la population de lymphocytes T FOXP3-positifs acquise sur la base du résultat de mesure de l'échantillon témoin.

7. Procédé de détection de lymphocytes T régulateurs de type effecteur selon l'une quelconque des revendications 4 à 6, dans lequel quand la population de lymphocytes T CD4-positifs acquise sur la base du résultat de mesure de l'échantillon témoin est représentée dans un histogramme avec un axe de niveau de FOXP3, une population de cellules présentant un niveau de FOXP3 supérieur ou égal à un niveau de FOXP3 à un point final d'une forme d'onde de crête d'un côté FOXP3-négatif de l'histogramme est spécifiée comme étant la population de lymphocytes T FOXP3-positifs.

8. Procédé de détection de lymphocytes T régulateurs de type effecteur selon la revendication 7, dans lequel quand une courbe approchée est appliquée à la forme d'onde de crête du côté FOXP3-négatif de l'histogramme, un point où la courbe approchée croise l'axe d'un côté de valeur plus élevée du niveau de FOXP3 est défini comme étant le point final.

9. Procédé de détection de lymphocytes T régulateurs de type effecteur selon la revendication 2 ou 3, dans lequel la valeur de référence est acquise en appliquant le rapport à une population CD45RA-négative parmi la population de lymphocytes T FOXP3-positifs acquise sur la base du résultat de mesure de l'échantillon témoin.

10. Procédé de détection de lymphocytes T régulateurs de type effecteur selon la revendication 9, dans lequel le rapport est établi de sorte qu'une proportion d'un nombre de cellules présentant un niveau de FOXP3 plus élevé par rapport à un nombre total de cellules de la population de lymphocytes T FOXP3-positifs acquise sur la base du résultat de mesure de l'échantillon témoin est de 62 % ou plus à 72 % ou moins.

11. Procédé de détection de lymphocytes T régulateurs de type effecteur selon la revendication 9, dans lequel le rapport est établi de sorte que le rapport d'un nombre de cellules présentant un niveau de FOXP3 plus élevé par rapport à un nombre de cellules présentant un niveau de FOXP3 plus bas est de 1,62 ou plus à 2,47 ou moins dans la population de lymphocytes T FOXP3-positifs acquise sur la base du résultat de mesure de l'échantillon témoin.

12. Procédé de détection de lymphocytes T régulateurs de type effecteur selon l'une quelconque des revendications 9 à 11, dans lequel quand la population de cellules CD45RA-négatives acquise sur la base du résultat de mesure de l'échantillon témoin est représentée dans un histogramme avec un axe du niveau de FOXP3, une population de cellules présentant un niveau de FOXP3 supérieur ou égal à un niveau de FOXP3 à un point final d'une forme d'onde de crête d'un côté FOXP3 négatif de l'histogramme est spécifiée comme étant la population de lymphocytes T FOXP3-positifs.

13. Procédé de détection de lymphocytes T régulateurs de type effecteur selon la revendication 12, dans lequel quand une courbe approchée est appliquée à la forme d'onde de crête du côté FOXP3-négatif de l'histogramme, un point où la courbe approchée croise l'axe d'un côté de valeur plus élevée du niveau de FOXP3 est défini comme étant le point final.

14. Procédé de détection de lymphocytes T régulateurs de type effecteur selon l'une quelconque des revendications 1 à 13, dans lequel, sur la base du résultat de mesure de l'échantillon biologique, une population de cellules qui est CD45RA-négative et présente un niveau de FOXP3 supérieur à la valeur de référence est détectée comme étant les lymphocytes T régulateurs de type effecteur.

15. Procédé de détection de lymphocytes T régulateurs de type effecteur selon la revendication 14, dans lequel, sur la base du résultat de mesure de l'échantillon biologique,
une seule population de lymphocytes vivants est extraite ;
une population de cellules CD3-positives est extraite à partir de la population de lymphocytes extraite ;
une population de cellules CD4-positives est extraite à partir de la population de cellules CD3-positives extraite ; et
une population de cellules qui est CD45RA-négative et présente un niveau de FOXP3 supérieur à la valeur de référence parmi la population de cellules CD4-positives extraite, est détectée en tant que les lymphocytes T régulateurs de type effecteur.

16. Procédé de détection de lymphocytes T régulateurs de type effecteur selon l'une quelconque des revendications 1 à 15, dans lequel l'échantillon témoin est un échantillon contenant un sang périphérique d'un humain en bonne santé, ou des cellules mononucléaires de sang périphérique purifiées à partir d'un sang périphérique d'un humain en bonne santé.

17. Dispositif d'analyse de lymphocytes T régulateurs de type effecteur utilisant la cytométrie en flux, le dispositif d'analyse de lymphocytes T régulateurs de type effecteur comprenant :
une section de mesure pour mesurer un échantillon biologique contenant une population de lymphocytes T CD4-positifs ; et
une section de commande qui est configurée, sur la base d'un résultat de mesure d'un échantillon témoin contenant une population de lymphocytes T CD4-positifs par la section de mesure, pour acquérir un niveau de FOXP3 qui est une référence de division pour diviser une population de lymphocytes T FOXP3-positifs contenue dans la population de lymphocytes T CD4-positifs à un rapport prédéterminé en tant que valeur de référence à utiliser pour mesurer l'échantillon biologique, et
sur la base d'un résultat de mesure de l'échantillon biologique contenant la population de lymphocytes T CD4-positifs par la section de mesure, pour détecter des lymphocytes T régulateurs de type effecteur parmi une population de cellules présentant un niveau de FOXP3 supérieur à la valeur de référence.

18. Système d'analyse de lymphocytes T régulateurs de type effecteur comprenant :
le dispositif d'analyse selon la revendication 17 ; et
un dispositif de marquage pour marquer par fluorescence des substances de test dans l'échantillon témoin et l'échantillon biologique.
